(19) Europäisches Patentamt / European Patent Office / Office européen des brevets

(11) **EP 4 140 035 B1**

(12) **EUROPEAN PATENT SPECIFICATION**

(45) Date of publication and mention
of the grant of the patent:
**04.12.2024  Bulletin 2024/49**

(21) Application number: **20737533.8**

(22) Date of filing: **24.04.2020**

(51) International Patent Classification (IPC):
*H03K 7/08* (2006.01)     *A61N 1/02* (2006.01)
*A61N 1/40* (2006.01)     *A61N 2/00* (2006.01)
*A61N 2/02* (2006.01)     *A61N 5/06* (2006.01)

(52) Cooperative Patent Classification (CPC):
**H03K 7/08; A61N 2/02; A61N 5/0622;** A61N 1/40;
A61N 2/002; A61N 2005/0648

(86) International application number:
**PCT/IB2020/053885**

(87) International publication number:
**WO 2021/214526 (28.10.2021 Gazette 2021/43)**

(54) **SYSTEM FOR THERAPEUTIC TREATMENTS WITH ELECTROMAGNETIC WAVES**

SYSTEM FÜR THERAPEUTISCHE BEHANDLUNGEN MIT ELEKTROMAGNETISCHEN WELLEN

SYSTÈME POUR TRAITEMENTS THÉRAPEUTIQUES AU MOYEN D'ONDES ÉLECTROMAGNÉTIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**01.03.2023  Bulletin 2023/09**

(73) Proprietor: **Thereson Holding Ltd
London E1 7LE (GB)**

(72) Inventor: **CONTI, Alberto Angelo
20123 Milano (IT)**

(74) Representative: **Buzzi, Notaro & Antonielli d'Oulx
S.p.A.
Corso Vittorio Emanuele II, 6
10123 Torino (IT)**

(56) References cited:
**WO-A1-2012/172504     US-A1- 2007 067 004**

## Description

### Technical field

The present disclosure relates to a system for therapeutic treatments with electromagnetic waves.

### Background

European patent application No. EP 3160582 A1 and International patent application No. WO 2012/172504 A1, describe systems that are able to subject various types of biological tissues and/or organs, usually of a mammal, typically a human, to an electromagnetic stimulus that drives into resonance the cell structures of the aforesaid tissues and organs. For example, as also described in European patent application No. EP 3160582 A1, it appears that different tissues and organs respond, *in vivo,* to frequencies of weak electromagnetic fields that have the property of sending specific cell structures of those tissues or organs into resonance. The characteristic frequencies are, for example, those of the electroencephalogram, which have a frequency range that falls between 0.1 and 42 Hz. Likewise, also other organs, tissues, and cell structures have typical frequency ranges, which have the property of responding to "resonance stimuli", if exposed to electromagnetic fields pulsating at these frequencies. For example, typically the electromagnetic field may be in a range between 40 nT and 100 $\mu$T. Moreover, in addition to resonance phenomena on organs, apparatus and tissues, effects on the cells that constitute them and in particular on the cell membrane may be observed. For example, it has been suggested that the electromagnetic fields applied to the cells are able to orient the water molecules, widely present in them, in resonance to the frequency of the electromagnetic field generated by the antennas. This orientation causes a local modification of the electrochemical gradient which in turn modifies the Van Der Waals forces that regulate the plasticity of the membrane lipids, as well as the ionic pumps (Sodium, Calcium, Potassium, etc.). Therefore, by stimulating the cellular systems at appropriate frequencies, it is possible to improve the functioning of the whole cell, including the respiration activities (exchange of oxygen), the production of energy (mitochondria), the protein synthesis and even the DNA replication.

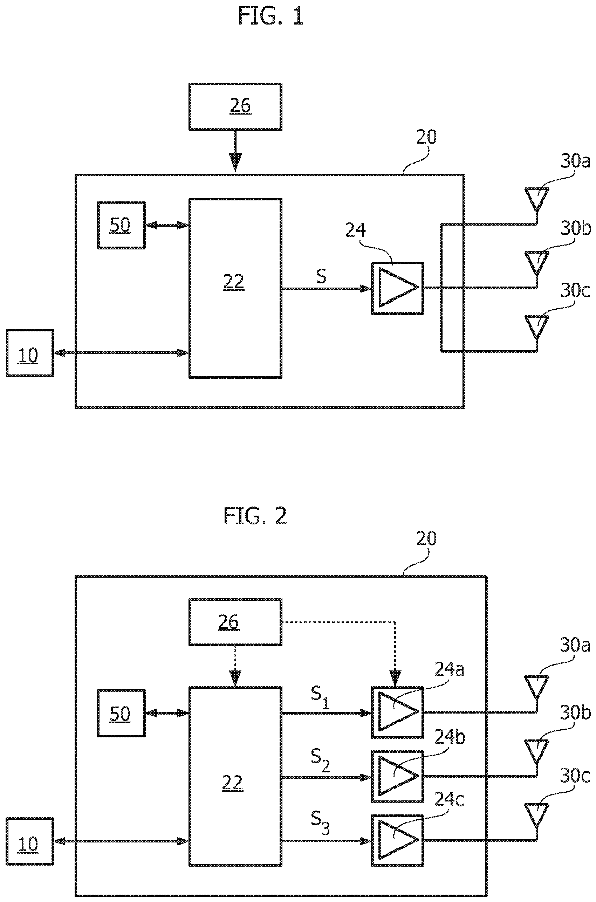

Figure 1 shows an example of a system for therapeutic treatments. In the example considered, the system comprises an apparatus 20 that generates at least one drive signal for at least one antenna or diffuser 30, which emits a corresponding electromagnetic treatment signal. In particular, in the example considered, the apparatus 20 comprises a control circuit 22 configured to generate a signal S that is sent through a power amplifier 24 to at least one antenna 30. For instance, in the example considered, three antennas 30a, 30b, and 30c are illustrated. Typically, the number of the antennas 30 is between one and twenty. As mentioned previously, typically the electromagnetic field emitted by an antenna 30 is between 40 nT and 100 $\mu$T.

In general, the antennas 30 are built for specific applications or treatments and may be in different forms, such as for example in the form of pads or mats, which may be plane or modeled anatomically to reproduce the area of application, or hand-pieces, thereby permitting a global or a local application of the electromagnetic field. Typically, the above diffusers/antennas are obtained via plane solenoids that are applied on a support. For instance, the diffusers illustrated in Figures 6 and 7 of document WO 2012/172504 A1 may be used for this purpose. A possible process for producing such antennas 30 is disclosed in European patent application No. EP 3 115 999 A1.

Specifically, in the example illustrated in Figure 1, the control circuit 22 generates only a single drive signal S, which is sent by means of an amplifier 24 to one or more antennas 30; *i.e.,* the antennas 30 are driven with the same signal S. In general, also a plurality of amplifiers 24 may be provided, for example one amplifier could be provided for each antenna 30, and each amplifier 24 could be set with a different amplification coefficient. Instead, Figure 2 shows an example, in which the control circuit 22 generates a plurality of driving signals S. For instance, in the example considered, the control circuit 22 generates three drive signals $S_1$, $S_2$ and $S_3$ that are sent through respective amplifiers 24a, 24b, and 24c to three diffusers 30a, 30b, 30c; *i.e.,* each antenna 30 is driven by a respective driving signal S and via a respective amplifier 24. In general, the arrangements illustrated in Figures 1 and 2 may also be combined in such a way that a plurality of antennas 30 is driven via at least one drive signal S, where at least one amplifier 24 is provided for each drive signal S and where each amplifier 24 can have a fixed or configurable amplification coefficient. For instance, in this way, a first drive signal $S_1$ may be used for a stimulation with systemic effect. For this purpose, an antenna 30 may be used, which is designed to emit electromagnetic radiation on a particular system of the person treated, such as for example the endocrine system, the immune system, the lymphatic system, or the muscular system. Instead, a second drive signal $S_2$ may be used for a stimulation with local effect. For this purpose, the first signal $S_1$ may comprise the characteristic frequencies of the system to be treated, whereas the second signal $S_2$ may comprise the characteristic frequencies of the local area to be treated, for example of the tissue or organ.

In Figures 1 and 2 is moreover shown a block 26, which represents a power supply circuit, such as for example a DC/DC or AC/DC electronic converter and/or a battery. In general, this power supply circuit 26 may be incorporated in the apparatus 20 (see Figure 2) or be provided at least in part externally (see Figure 1). The apparatus 20 may comprise a user interface 50, which includes, for example, a plurality of keys and/or state indicators, a touch screen, etc. The system may also comprise an external processing unit 10, such as for example a PC, which is asrranged to communicate with the apparatus 20, for instance for configuring the apparatus 20 and/or for downloading a treatment protocol from the apparatus 20. Generally, any suitable communication interface may be used for exchanging data between the control circuit 22 and the processing circuit 10. For instance, for this purpose the apparatus 20 may comprises a wired or wireless communication interface, or a reader device for reading and/or writing a portable memory, such as a memory card.

According to document WO 2012/172504 A1, in order to determine a stimulus that is positive for tissue repair, it is necessary to obtain simultaneously resonance effects from the different organs/tissues involved. To be able to create simultaneously such well-defined frequencies only within certain ranges, document WO 2012/172504 A1 proposes an apparatus that generates an electromagnetic wave with specific contributions in frequency that is constituted by a cascade of base pulses I generated at a certain frequency.

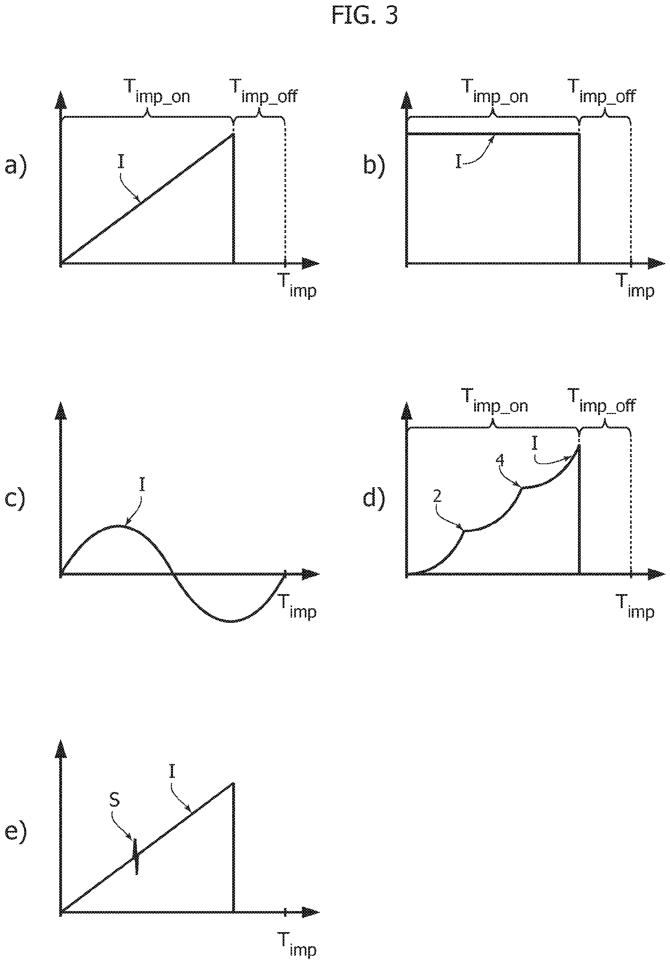

Figure 3 illustrates possible waveforms of such a base pulse I. For example, the base pulse I can be a sawtooth or triangular waveform (Figure 3a), a square waveform (Figure 3b), or a sinusoidal waveform (Figure 3c). Generally, the base pulse I may also have other waveforms. For example, Figure 3d shows a base pulse I comprising a series of three curved profiles in such a way that the waveform is increasing and comprises two cusps 2 and 4. According to document WO 2012/172504 A1, the base pulse I may have a frequency $f_{imp}$ ($f_{imp}$ = 1/$T_{imp}$) in a range between 100 Hz and 1 kHz, preferably between 100 and 400 Hz, even more preferably between 150 and 250 Hz. For example, preferably the base pulse I has a frequency $f_{imp}$ of 190 Hz (in particular 189.75 Hz), 213 Hz (in particular 212.76 Hz) or 231 Hz (in particular 231.48 Hz). As shown in Figures 3a, 3b and 3c, a pulse-width modulation (PWM) may optionally be applied to the base pulse I with a switching period $T_{imp}$. Consequently, the pulse is activated for a duration $T_{imp\_on}$ and the pulse is deactivated for a duration $T_{imp\_off}$ (with $T_{imp\_off}$ = $T_{imp}$ - $T_{imp\_on}$), where the durations $T_{imp\_on}$ and $T_{imp\_off}$ can be varied in the interval ] 0; $T_{imp}$[.

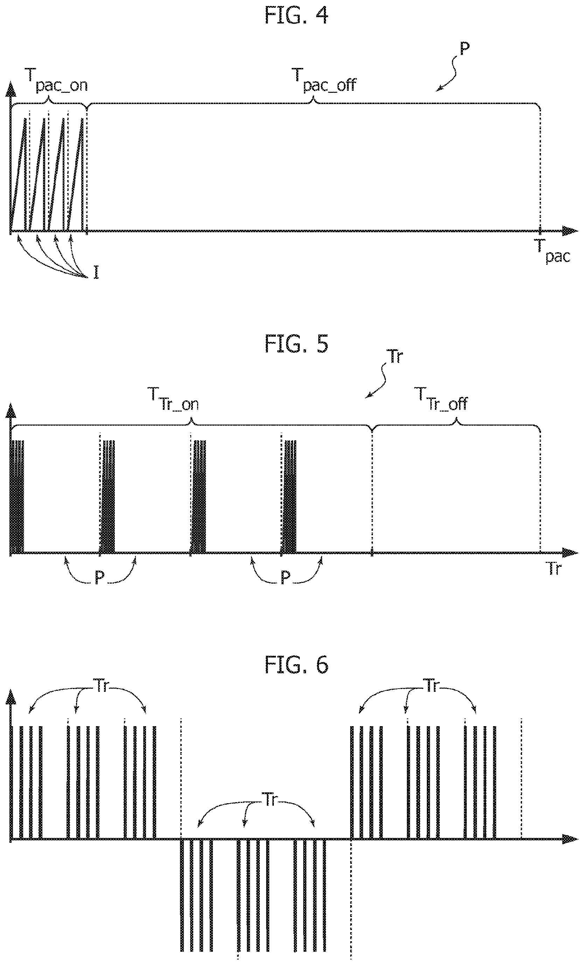

According to document WO 2012/172504 A1, a given number $n_{imp}$ of these base pulses I are grouped together to generate a packet P, i.e., the base pulses I within a packet P with a duration $T_{pac}$ ($T_{pac}$ = 1/$f_{pac}$) are activated for a duration $T_{pac\_on}$ (corresponding to a multiple of the duration $T_{imp}$) and deactivated for a remaining duration $T_{pac\_off}$ ($T_{pac\_off}$ = $T_{pac}$ - $T_{pac\_on}$). For example, Figure 4 shows an example of a packet P comprising four base pulses I. The duration of the packet $T_{pac}$ may be, e.g., between 5 ms and 2 s, preferably between 20 ms and 1 s, even more preferably between 25 ms and 500 ms.

According to document WO 2012/172504 A1, a given number $n_{pac}$ of these packets P are grouped to generate a train of packets Tr, i.e., the packets P within a train Tr with a duration $T_{tr}$ ($T_{tr}$ = 1/$f_{tr}$) are activated for a duration $T_{tr\_on}$ (corresponding to a multiple of the duration $T_{pac}$) and deactivated for a remaining duration $T_{tr\_off}$ ($T_{tr\_off}$ = $T_{tr}$ - $T_{tr\_on}$). For example, Figure 5 shows an embodiment of a train Tr comprising four packets P. The duration of the train $T_{tr}$ may be, e.g., between 25 ms and 10 s, preferably between 100 ms and 5 s, even more preferably between 1 and 5 s.

Thus, a series of identical packets P are generated by the apparatus of document WO 2012/172504 A1, wherein each packet P comprises a series of base pulses I. Specifically, in the case where the base pulses I were always activated (i.e., $T_{pac\_off}$ = 0 and $T_{tr\_off}$ = 0), the spectrum of the signal would contain only the spectrum of the base pulse I. For example, in the case of a base pulse I with sinusoidal waveform at 212.76 Hz, the spectrum would contain a single peak at 212.76 Hz. However, due to the fact that the periodic signal is truncated remaining defined only within a certain interval of definition, the resulting spectrum is broadened in the frequency domain by a value equal to the inverse of the interval of definition of the signal itself. Consequently, the final signal also comprises the characteristics in frequency of the packets P and of the trains T, i.e., the harmonics for the frequencies $f_{pac}$ and $f_{tr}$. Substantially, in this way it is possible to define via the frequency $f_{tr}$ a minimum frequency and via the frequency $f_{pac}$ a maximum frequency. Consequently, the apparatus 20 stimulates the cells not via the fundamental harmonics of the base pulse I but via the secondary harmonics resulting in the interval between $f_{tr}$ and $f_{pac}$.

For example, document WO 2012/172504 A1 discloses that the apparatus may comprise the following treatment programs, which can be present also individually or in groups for carrying out a specific therapeutic treatment:

1) program 1: saw-tooth base pulse with frequency $f_{imp}$ = 212.76 Hz (see Figure 1a), where the duration of the base pulse is $T_{imp}$ = 4.7 ms, the number of base pulses in a pulse packet is 44, the pause between the packets is $T_{pac\_off}$ = 140 ms, the number of pulse packets in a pulse train is 4, and the pause between the trains is $T_{tr\_off}$ = 1450 ms;

2) program 2: saw-tooth base pulse with frequency $f_{imp}$ = 231.48 Hz, where the duration of the base pulse is $T_{imp}$ = 4.32 ms, the number of base pulses in a pulse packet is 34, the pause between the packets is $T_{pac\_off}$ = 105 ms, the number of pulse packets in a pulse train is 4, and the pause between the trains is $T_{tr\_off}$ = 1100 ms;

3) program 3: saw-tooth base pulse with frequency $f_{imp}$ = 212.76 Hz, where the duration of the base pulse is $T_{imp}$ = 4.7 ms, the number of base pulses in a pulse packet is 20, the pause between the packets is $T_{pac\_off}$ = 55 ms, the number of pulse packets in a pulse train is 4, and the pause between the trains is $T_{tr\_off}$ = 600 ms;

4) program 4: saw-tooth base pulse with frequency $f_{imp}$ = 231.48 Hz, where the duration of the base pulse is $T_{imp}$

= 4.32 ms, the number of base pulses in a pulse packet is 18, the pause between the packets is $T_{pac\_off}$ = 37 ms, the number of pulse packets in a pulse train is 4, and the pause between the trains is $T_{tr\_off}$ = 480 ms;

5) program 5: saw-tooth base pulse with frequency $f_{imp}$ = 231.48 Hz, where the duration of the base pulse is $T_{imp}$ = 4.32 ms, the number of base pulses in a pulse packet is 16, the pause between the packets is $T_{pac\_off}$ = 24 ms, the number of pulse packets in a pulse train is 4, and the pause between the trains is $T_{tr\_off}$ = 380 ms;

6) program 6: saw-tooth base pulse with frequency $f_{imp}$ = 231.48 Hz, where the duration of the base pulse is $T_{imp}$ = 4.32 ms, the number of base pulses in a pulse packet is 14, the pause between the packets is $T_{pac\_off}$ = 16 ms, the number of pulse packets in a pulse train is 4, and the pause between the trains is $T_{tr\_off}$ = 350 ms;

7) program 7: saw-tooth base pulse with frequency $f_{imp}$ = 212.76 Hz, where the duration of the base pulse is $T_{imp}$ = 4.7 ms, the number of base pulses in a pulse packet is 12, the pause between the packets is $T_{pac\_off}$ = 6.6 ms, the number of pulse packets in a pulse train is 4, and the pause between the trains is $T_{tr\_off}$ = 220 ms;

8) program 8: saw-tooth base pulse with frequency $f_{imp}$ = 231.48 Hz, where the duration of the base pulse is $T_{imp}$ = 4.32 ms, the number of base pulses in a pulse packet is 10, the pause between the packets is $T_{pac\_off}$ = 2.55 ms, the number of pulse packets in a pulse train is 4, and the pause between the trains is $T_{tr\_off}$ = 176 ms; and

9) program 9: base pulse with cusps with frequency $f_{imp}$ = 189.75 Hz (see Figure 1d), where the duration of the base pulse is $T_{imp}$ = 5.27 ms, the number of base pulses in a pulse packet is 5, the pause between the packets is $T_{pac\_off}$ = 36 ms, the number of pulse packets in a pulse train is 20, and the pause between the trains is $T_{tr\_off}$ = 3000 ms.

For example, the timing in [ms] of the above programs may be summarized according to the following table:

| # | $T_{imp}$ | $n_{imp}$ | $T_{pac\_on}$ | $T_{pac\_off}$ | $T_{pac}$ | $n_{pac}$ | $T_{tr\_on}$ | $T_{tr\_off}$ | $T_{tr}$ |
|---|---|---|---|---|---|---|---|---|---|
| 1 | 4.70 | 44 | 206.80 | 140.00 | 346.80 | 4 | 1387.20 | 1450 | 2837.20 |
| 2 | 4.32 | 34 | 146.88 | 105.00 | 251.88 | 4 | 1007.52 | 1100 | 2107.52 |
| 3 | 4.70 | 20 | 94.00 | 55.00 | 149.00 | 4 | 596.00 | 600 | 1196.00 |
| 4 | 4.32 | 18 | 77.76 | 37.00 | 114.76 | 4 | 459.04 | 480 | 939.04 |
| 5 | 4.32 | 16 | 69.12 | 24.00 | 93.12 | 4 | 372.48 | 380 | 752.48 |
| 6 | 4.32 | 14 | 60.48 | 16.00 | 76.48 | 4 | 305.92 | 350 | 655.92 |
| 7 | 4.70 | 12 | 56.40 | 6.60 | 63.00 | 4 | 252.00 | 220 | 472.00 |
| 8 | 4.32 | 10 | 43.20 | 2.55 | 45.75 | 4 | 183.00 | 176 | 359.00 |
| 9 | 5.27 | 5 | 26.35 | 36.00 | 62.35 | 20 | 1247.00 | 3000 | 4247.00 |

For example, document WO 2012/172504 A1 discloses that the duration of the treatment for the above programs may be 480 s. Document WO 2012/172504 A1 also discloses that this process of construction of the signal can be extended to further levels beyond the trains (*i.e.,* constructing "sets of trains" and so forth, with a construction at progressively higher levels) in the case where it were desired to obtain a further capacity of regulation of the frequency contents within specific and pre-determined ranges. For example, Figure 6 shows an example in which three trains Tr are grouped to form a set of trains. Figure 6 shows also that the polarity of the set of trains can be alternated, *i.e.,* reversed for each successive set of trains. For example, document WO 2012/172504 A1 discloses that the polarity of the programs 1 to 8 may be reversed every 120 s, whilst the polarity of the program 9 may be reversed every 180 s.

The frequency content of these pulses, packets, and trains is hence equivalent to obtaining the resonance frequencies, and those alone, that characterize the typical range of the various organs/tissues. Moreover, this structure of the signal enables not only the desired frequencies to be obtained and only those, but also enables modification thereof on one and the same apparatus in a simple and deterministic way, adjusting the typical parameters of the components of the signal. Generally, the base pulses I may also contain some spikes, which guarantee that in addition to the main frequency there is an adequate content of additional harmonics. For example, Figure 3e shows an embodiment of a base pulse I having a sawtooth shape that comprises a spike S. For example, document WO 2012/172504 A1 discloses that the above-mentioned programs may stimulate the parts of the body and/or generate the effects in a human body as listed below:

1) program 1: central nervous system (CNS), limiting its function as far as creating sub-hypnotic states; ansiolytic, sedative, hypno-inducing effect;

2) program 2: paranasal sinuses and cranial sinuses, bronchia and respiratory tree, generalized organic stimulus; improvement of pulmonary ventilation;

3) program 3: CNS and peripheral nervous system, stimulation, and stimulating and repairing effect;

4) program 4: neurovegetative system and correlated functions;

5) program 5: system of metabolization of endogenous and exogenous substances, liver, lungs, stomach; anti-inflammatory and disintoxicating effect in support of pharmacological therapies in progress and release of states of homotoxicological deposit;

6) program 6: artero-venous and lymphatic circulatory system;

7) program 7: synovial membranes, articular capsules, tendons, cartilage, and mediators involved in flogosis; anti-inflammatory and antalgic effect;

8) program 8: musculoskeletal apparatus and mediators involved in generation of pain, including the production of substance P; antalgic effect;

9) program 9: psychological system, neurological system, endocrine system, immunitary stimulation; regulating and cell-regenerating effect.

For example, document WO 2012/172504 A1 discloses that, for treating a diabetic foot, the apparatus 20 may use a sequence that comprises in order program 9, program 6, and program 8; namely, for the indicated duration of a program of 480 s, the duration of the entire treatment would be 1440 s.

Object and summary

Recent experiments conducted by the present applicant have demonstrated the effectiveness of the higher frequency programs 6 to 9. However, the lower frequency programs 1 to 4, while showing a positive result, seem to have a lower efficiency than expected. For example, when using programs 1 or 3 of the apparatus of document WO 2012/172504 A1 for the treatment of the central and/or peripheral nervous system, *e.g.* for treating chronic neuroinflammation and/or neurodegenerative diseases such as Alzheimer's disease, multiple sclerosis or Parkinson's disease, only minor improvements could be shown. Considering the foregoing, it is therefore an object of various embodiments to provide solutions which improve the treatment efficiency of the apparatus of document WO 2012/172504 A1.

According to one or more embodiments, the above object is achieved by a system for therapeutic treatments with electromagnetic waves having the distinctive elements set forth specifically in the ensuing claims. The claims form an integral part of the technical teaching of the description provided herein.

As described in the foregoing, various embodiments of the present disclosure relate to a system for therapeutic treatments with electromagnetic waves, comprising an antenna and an apparatus configured to generate a supply current for the antenna in order to generate the electromagnetic wave.

Accordingly, various embodiments relate to a system for therapeutic treatments with electromagnetic waves. The system comprises an antenna 30 and an apparatus 20a configured to generate a supply current $i_{out}$ for the antenna 30 in order to generate the electromagnetic waves.

In various embodiments, the apparatus comprises a digital processing circuit configured to generate a first PWM signal having a given duty cycle, wherein the first PWM signal is set, for each switching cycle, to high for a switch-on period and to low for a switch-off period, a switching stage configured to generate an amplified PWM signal by amplifying the first PWM signal, and an analog low-pass or band-pass filter configured to generate the supply current by filtering the amplified PWM signal;

Specifically, in various embodiments, the digital processing circuit is configured to generate a sequence of first digital values of a periodic base pulse having a given frequency. Moreover, in various embodiments, the digital processing circuit is configured to generate an enable signal with a particular timing. For example, for this purpose, the digital processing circuit is configured to generate a second PWM signal, wherein the second PWM signal is set, for each switching cycle, to a respective first logic level for a packet switch-on period and to a respective second logic level for a packet switch-off period, and a third PWM signal, wherein the third PWM signal is set, for each switching cycle, to a respective first logic level for a train switch-on period and to a respective second logic level for a train switch-off period. In this case, the digital processing circuit is configured to set to the enable signal to a respective first logic level when the second PWM signal has the respective first logic level and the third PWM signal has the respective first logic level, and to a respective second logic level when the second PWM signal has the respective second logic level or the third PWM signal has the respective second logic level. In various embodiments, the digital processing circuit may then generate a second digital value, wherein the second digital value is set to the first digital value when the enable signal has the respective first logic level and to zero when the enable signal has the respective second logic level.

Specifically, in the embodiment considered, this second digital value represents a reference value for the supply current $i_{out}$ for the antenna 30. Accordingly an analog-digital converter could be used to supply the antenna as a function of the second digital value. Conversely, as mentioned before, in various embodiments, the digital processing circuit is configured to generate the first PWM signal, which is then amplified by the switching stage and filtered by the analog low-pass or band-pass filter. Accordingly, in various embodiments, the digital processing circuit is configured to generate the first PWM signal as a function of the second digital value. Specifically, in various embodiments, the apparatus comprises also a current sensor configured to provide a digital sample indicative of the amplitude of the supply current, and the digital processing circuit is configured to generate the first PWM signal as a function of a third digital value indicative of the given duty cycle, and vary this third digital value via a discrete proportional-integral regulation configured to regulate the difference between the second digital value and the digital sample to zero.

For example, in various embodiments, the digital processing circuit is implemented with an FPGA or an ASIC comprising a digital signal generator configured to generate the sequence of first digital values, a first digital PWM generator circuit configured to generate the first PWM signal as a function of the third digital value indicative of the given duty cycle, a second digital PWM generator circuit configured to generate the second PWM signal, a third digital PWM generator circuit configured to generate the third PWM signal, a first combinational logic circuit configured to generate the enable signal as a function of the second PWM signal and the third PWM signal, a second combinational logic circuit configured to generate the second digital value as a function of the first digital value and the enable signal, and a discrete proportional-integral regulator circuit configured to vary the third digital value as a function of the second digital value and the digital sample.

For example, in various embodiments, the periodic base pulses has a saw-tooth profile. In this case, the digital signal generator may comprise a counter configured to, in response to a clock signal having a given frequency, increase the first digital values by an increment value, and reset the first digital values when the first digital values reaches a given maximum value. In this case, the digital signal generator may also comprises an increment control circuit configured to generate the increment value for the counter as a function of data identifying the frequency or the clock signal, data identifying the frequency of the periodic base pulses, and the maximum value of the counter. Instead, for arbitrary base pulse waveforms, the digital signal generator may comprises a look-up table comprising a plurality of elements corresponding to a given number of samples of a standard waveform of the base pulses with a given standard frequency. Specifically, each element of the LUT has stored the amplitude of a respective sample of the standard waveform of the base pulses. Accordingly, the look-up table may receive at input a (phase) count value selecting a given sample, and the first digital value may be determined as a function of the amplitude of the respective selected sample read from the LUT. Accordingly, a (phase) counter may be configured to, in response to a clock signal, increase the (phase) count value by an increment value, whereby the counter represents a phase accumulator. In this case, an increment control circuit may be configured to generate the increment value as a function of data identifying the standard frequency of the standard waveform and data identifying the frequency of the periodic base pulses.

In various embodiments, in order to improve the precision of the frequency of the base pulse, such increment control circuits may be configured to calculate an internal increment value, wherein the internal increment value has an integer part having a number of bits corresponding to the number of bits of the increment value, and a fractional part, e.g. having between 4 and 16 bits, preferably between 8 and 12 bits. Accordingly, the increment control circuit may be configured to vary the increment value, such that the increment value has an average value corresponding to the (higher resolution) internal increment value.

In various embodiments, the apparatus may also be configured to invert the polarity of the supply current $i_{out}$. For example, in this case, the digital processing circuit comprises a third digital PWM generator circuit configured to generate a polarity signal indicating the polarity for the supply current $i_{out}$. For example, in this case, the switching stage may comprises a first half-bride comprising a first and a second electronic switch, wherein the intermediate node between the first and the second electronic switch is a first switching node, and a second half-bride comprising a third and a fourth electronic switch, wherein the intermediate node between the third and the fourth electronic switch is a second switching node, wherein the amplified PWM signal corresponds to the voltage between the first switching node and the second switching node. Accordingly, a driver circuit of the switching stage may be configured to generate drive signals for the first, second, third and fourth electronic switch as a function of the first PWM signal and the polarity signal.

In various embodiments, the apparatus may also comprise a further digital processing unit. For example, in various embodiments, the digital processing circuit comprises a communication interface for exchanging data with the further digital processing unit; and a control circuit configured to receive via the first communication interface data identifying a treatment program to be executed and determine, as a function of the data identifying a treatment program to be executed, the frequency of the periodic base pulse, the packet switch-on period and the packet switch-off period, and the train switch-on period and the train switch-off period. For example, the data identifying a treatment program to be executed may comprise a program number or the respective timing data. The data identifying the treatment program to be executed may also identify the duty cycle of a PWM modulation applied directly to each base pulse.

For example, the further digital processing unit may be configured to receive data identifying a treatment application, determine a sequence of a plurality of treatment programs as a function of the data identifying the treatment application and then execute sequentially the treatment programs of the sequence of treatment programs by sending data identifying the respective treatment program to the digital processing unit. For example, the further digital processing unit may be configured to receive the data identifying the treatment application via a user interface and/or a communication interface configured to exchange data with a processing system, such as a computer or smartphone. In various embodiments, the digital processing unit is an FPGA programmable via respective program data stored to a first non-volatile memory and/or the further digital processing unit is a microprocessor programmable via a respective firmware stored to a second non-volatile memory. In this case, the further digital processing unit may be configured to receive new program data for the digital processing unit and store the new program data to the first non-volatile memory, and/or receive a new firmware for the further digital processing unit and store the new firmware to the second non-volatile memory.

In various embodiments, the apparatus may be configured to generate one or more further signals, synchronized with the supply current $i_{out}$ for the antenna, such as an audio signal, *e.g.* applied to a headphone or speaker, a signal used to drive one or more light sources, and/or a signal used to drive a vibration transducer. For example, in various embodiments, the apparatus is configured to generate theses one or more further signals as a function of the second digital value.

Brief description of the annexed drawings

The embodiments of the present disclosure will now be described with reference to the annexed plates of drawings, which are provided purely to way of non-limiting example and in which:
The features and advantages of the present invention will become apparent from the following detailed description of practical embodiments thereof, shown by way of non-limiting example in the accompanying drawings, in which:

- Figures 1 and 2 show examples of systems for therapeutic treatments wherein electromagnetic waves are generated as a function of a drive signal comprising base pulses organized as packets and trains;
- Figures 3a to 3e show exemplary waveforms of the base pulses of the drive signal of Figures 1 and 2;
- Figure 4 shows an example of a pulse packet of the drive signal of Figures 1 and 2;
- Figure 5 shows an example of a train of packets of the drive signal of Figures 1 and 2;
- Figure 6 shows an example of the inversion of the polarity of the drive signal of Figures 1 and 2;
- Figure 7 shows a further example of a system for therapeutic treatments with electromagnetic waves;
- Figure 8 shows an embodiment of a drive signal according to the present disclosure;
- Figure 9 shows an embodiment of a system for therapeutic treatments with electromagnetic waves, wherein the system comprises an apparatus configured to generate the drive signal of Figure 8, wherein the apparatus comprises a first digital processing circuit, a second digital processing circuit and a power amplifier;
- Figure 10 shows a flow chart of the operation of the first digital processing circuit of the apparatus of Figure 9;
- Figures 11A and 11B show embodiments of a switching stage of the power amplifier of Figure 9;
- Figures 12A and 12B show embodiments of the drive signals of the switching stages of Figures 11A and 11B, respectively;
- Figure 13 shows an embodiment of the power amplifier of the apparatus of Figure 9 comprising a switching stage of Figure 11A or 11B;
- Figure 14 shows a block diagram of the second digital processing circuit of the apparatus of Figure 9;
- Figure 15 shows embodiments of various signals generated by the second digital processing circuit of Figure 14; and
- Figures 16 and 17 show embodiments of a waveform generator used by the second digital processing circuit of Figure 14.

Detailed description of embodiments

In the ensuing description, various specific details are illustrated aimed at enabling an in-depth understanding of the embodiments. The embodiments may be provided without one or more of the specific details, or with other methods, components, materials, etc. In other cases, known structures, materials, or operations are not shown or described in detail so that various aspects of the embodiments will not be obscured.

Reference to "an embodiment" or "one embodiment" in the framework of this description is meant to indicate that a particular configuration, structure, or characteristic described in relation to the embodiment is comprised in at least one embodiment. Hence, phrases such as "in an embodiment", "in one embodiment", or the like that may be present in various points of this description do not necessarily refer to one and the same embodiment. Moreover, particular conformations, structures, or characteristics may be combined in any adequate way in one or more embodiments.

The references used herein are only provided for convenience and hence do not define the sphere of protection or the scope of the embodiments.

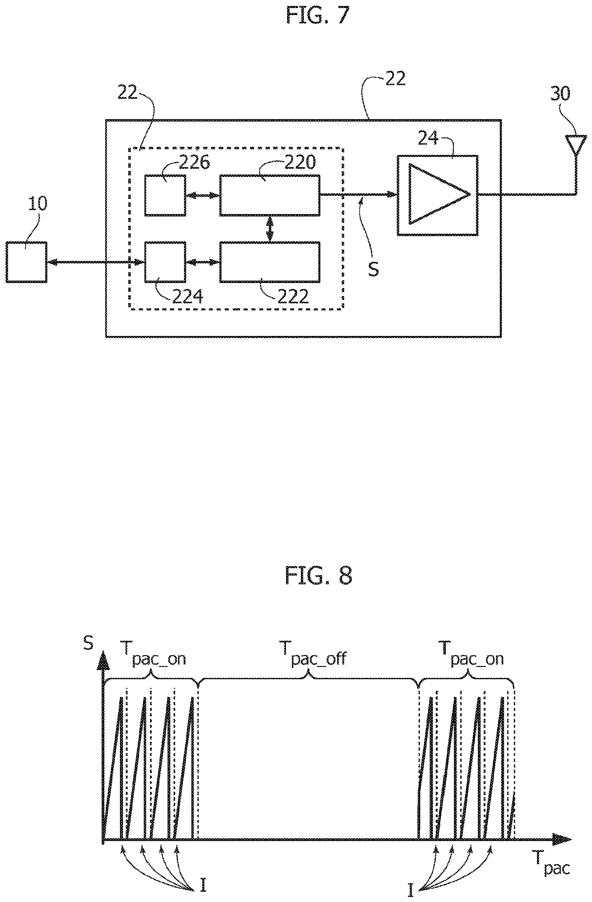

In Figures 7 to 17 described below, parts, elements or components that have already been described with reference to Figures 1 to 6 are designated by the same references used previously in these figures. The description of these elements has already been made and will not be repeated in what follows in order not to burden the present detailed description.

As explained in the foregoing, various embodiments of the present description relate to a system for therapeutic treatments with electromagnetic waves. As described in the foregoing, when using programs 1 or 3 of the apparatus of document WO 2012/172504 A1 for the treatment of the central and/or peripheral nervous system, *e.g.* for treating chronic neuroinflammation and/or neurodegenerative diseases such as Alzheimer's disease, multiple sclerosis or Parkinson's disease, only minor improvements could be shown.

Generally, the inventor has observed that this problem could have various reasons, such as incorrect frequencies of the programs, or that a single program is insufficient for the treatment of a particular decease. In this respect, the inventor of the present application has observed, that the combination of programs 1, 3 and 6 seems to be most suitable for the treatment of the central and/or peripheral nervous system, *e.g.* for the treatment of the above mentioned diseases, insofar as an improvement of the result of the treatment could be observed. However, a rather surprising improvement could be observed when slightly adapting the embodiment shown in Figure 5 of document WO 2012/172504 A1, and which is reproduced herein as Figure 7. Specifically, in the embodiment shown in Figure 7, the apparatus 20 comprises a control circuit 22 configured for generating a signal S that corresponds to the signal described previously. In the embodiment considered, the signal S is sent through a power amplifier 24 to an antenna 30.

Specifically, according to document WO 2012/172504 A1, the signal S comprises a plurality of base pulses I grouped in pulse packets P and in pulse trains Tr, wherein each pulse packet P consists of a series of base pulses I followed by a first pause $T_{pac\_off}$, and wherein each pulse train Tr consists of a series of pulse packets P followed by a second pause $T_{tr\_off}$. For example, each base pulse I may have a saw-tooth, square-wave, or sinusoidal waveform; or each base pulse I may comprise a series of curved profiles in such a way that in a pulse time interval $T_{imp\_on}$ the waveform is increasing and comprises a plurality of cusps 2 and 4.

In the embodiment considered, the control circuit 22 comprises a waveform generator 226 configured to generate different waveforms (see for example Figure 3) with a certain frequency $f_{imp}$. In the embodiment considered, the control circuit 22 comprises also a processing unit 220 configured to generate the signal S. In the embodiment considered, the control circuit 22 comprises also a memory 222 in which the characteristic data of the signal S are saved, such as for example data identifying the durations $T_{pac\_on}$, $T_{pac\_off}$, $T_{tr\_on}$ and $T_{tr\_off}$. In the case also the base pulses I are configurable, the memory 222 may also store data identifying the respective waveform and/or the durations $T_{imp\_on}$ and $T_{imp\_off}$ (see Figure 3).

As described in the foregoing, document WO 2012/172504 A1 generates a signal having trains Tr of identical packets P, each comprising a given number $n_{imp}$ of base pulses I. This is also shown in Figures 4 and 5, where the base pulses I start with the beginning of a packet P and all packets P are identical, thereby permitting that precise and constant harmonics are generated.

Accordingly, by using a waveform generator 226, such a sequence of trains Tr of packets P may e obtained by:

- activating the waveform generator 226 at the beginning of a packet P and deactivating the waveform generator 226 at the end of the interval $T_{pac\_on}$, or, *e.g.* in case a PWM modulation of the base pulse I is performed, activating the waveform generator 226 at the beginning of each interval $T_{imp}$ and deactivating the waveform generator 226 at the end of the interval $T_{imp\_on}$; or
- by using switch-off durations $T_{pac\_off}$ and $T_{tr\_off}$, which are multiples of the time $T_{imp}$ of the base pulse I, whereby the start of a packet intrinsically corresponds to the start of a base pulse I.

Generally, the second option may be rather unfeasible, because the switch-off durations $T_{pac\_off}$ and $T_{tr\_off}$ are not necessarily multiples of the time $T_{imp}$. Accordingly, in the embodiment of document WO 2012/172504 A1, the control circuit 22 uses the first option, wherein the processing unit 220 is configured to activate and deactivate the signal provided by the waveform generator 226 according to the data identifying durations $T_{pac\_on}$, $T_{pac\_off}$, $T_{tr\_on}$ and $T_{tr\_off}$, and possibly also of the durations $T_{imp\_on}$ and $T_{imp\_off}$ (as stored to the memory 222). However, the inventor has observed that the result of the treatment improves significantly for the low frequency programs 1 to 4 when the waveform generator 226 is not activated at the beginning of each packet P, or even each interval $T_{imp}$, but the waveform generator 226 is maintained running and provides at output continuous base pulses I, with a given frequency $f_{imp}$ / a given period $T_{imp}$. As mentioned before, while the frequency $f_{imp}$ / period $T_{imp}$ is usually constant for a given treatment program 1 to 9, the frequency $f_{imp}$ / period $T_{imp}$ may still be settable, *e.g.* for the frequencies 213 Hz and 231 Hz mentioned before.

As mentioned before, for the specific programs 1 to 9 mentioned before, the period $T_{pac}$ of a packet P does not correspond to a multiple of the period $T_{imp}$ of the base pulse I. Accordingly, as also shown in Figure 8, a shifting effect is introduced between the packets P of a sequence of packets P. Thus, also in this case, the duration $T_{pac\_on}$ corresponds to a multiple of the period $T_{imp}$, but the packet P usually do not consist in a series of identical base pulses I, because the first pulse starts usually at an intermediate position of the base pulse I and similarly the last pulse usually ends at an intermediate position of the base pulse I. Thus, in such an arrangement, the packets P are not identical, and the spectrum of the signal S is broadened around the harmonics, more or less as in a dithering operation of the signal S. A similar effect occurs also for the trains, because the period $T_{tr}$ of a train Tr does not correspond to a multiple of the period $T_{imp}$ of the base pulse I.

As mentioned before, such a broadening of the spectrum would be undesirable according to document WO 2012/172504 A1, which provides an arrangement configured to generate clearly defined series of frequencies only within certain ranges, while avoiding a wider frequency ranges, which according to document WO 2012/172504 A1 would provide only a low probability of inducing the effect of therapeutic resonance. In this respect, document EP 3160582 A1 already mentioned that the dynamic variation of the harmonics could be useful, *e.g.* in order to prevent the phenomenon of cell adaptation, with reduction of the sensitivity to the stimulation induced and of the consequent responses. Accordingly, the inventor performed further experiments, in particular:

- varying the frequency $f_{imp}$ of the base pulse, and the switch-off durations $T_{pac\_off}$ and $T_{tr\_off}$ of the packets in the apparatus of document WO 2012/172504 A1 according to a random dithering operation; and
- varying the frequencies of the sinusoidal signals of the apparatus of document EP 3160582 A1 according to a random dithering operation.

However, surprisingly the above described shifting operation by activating/deactivating a continuously running base pulse I obtained the best results. A possible explanation may reside in the fact that a dithering operation results in a random and rather chaotic variation of the frequencies of the electromagnetic wave. Conversely, the shift operation maintains the frequency of the trains, but additional and variable low power harmonics of the truncated first and last packet P, and similarly the first and last base pulses are added. Moreover, the shifting operation is not casual, but essentially results in a repetitive and cyclic pattern, which seems to be useful for the resonance effects.

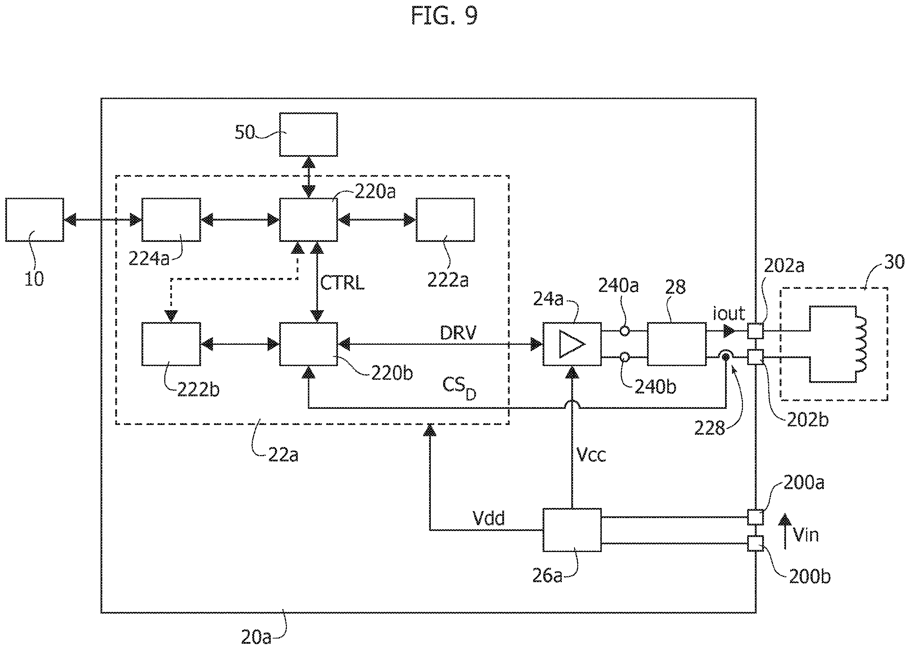

Figure 9 shows an embodiment of an apparatus 20a according to the present description. Specifically, the general architecture corresponds the architecture already described with respect to Figures 1 and 2, and the respective description fully applies. Specifically, also in this case, the apparatus 22a comprises a power amplifier, and a control circuit 22a configured to generate a drive signal DRV for the power amplifier. Specifically, as will be described in greater detail in the following, in various embodiments, the power amplifier is a class D power amplifier comprising a power/switching stage 24a receiving at input a PWM signal switching between two logic levels, *e.g.* a voltage Vdd and ground. For example, the voltage Vdd may correspond to the supply voltage of the control circuit 22a, such as a voltage between 2 and 5 V. In a class D amplifier, the power/switching stage provides thus at output terminals 240a and 240b an amplified PWM signal, *e.g.* switching between a voltage Vcc and ground, with Vcc being greater than the voltage Vdd, or between Vcc and -Vcc. For example, the voltage Vss may be between 9 and 48 V. In a class D amplifier, the amplified PWM signal at the output 240a/240b of the power stage 24a is provided to a filter stage 28, such as a lowpass or band-pass filter, which thus provides at output a signal having an amplitude proportional to the duty cycle of the amplified PWM signal. Accordingly, the signal at the output of the filter stage 28 may be provided to an antenna 30. For example, as shown in Figure 9, the apparatus 20a may comprise for this purpose two terminals 202a and 202b configured to be connected to an antenna/diffuser 30.

Generally, the filter stage 28 may also be external with respect to the apparatus 20a and, *e.g.*, incorporated in the antenna/diffuser 30. Generally, the filter stage 28 may also comprise the capacitance and inductance of the antenna 30. Accordingly, the terminals 202a and 202b may be connected to the output of the filter stage 28 (when the filter stage 28 is incorporated in the apparatus 20a) or to the output of the power stage 24a (when the filter stage 28 is external with respect to the apparatus 20a).

Accordingly, the apparatus 20a may also comprise a power supply 26a configured to receive an input voltage Vin and generate the supply voltage Vdd (and possible further supply voltages) for the control circuit 22a and the supply voltage Vcc for the power stage 24a. Generally, the power supply 26a may be any AC/DC or DC/DC power supply adapted to generate a plurality of supply voltages (*e.g.* from the mains and/or a battery integrated in the apparatus 20a). For example, in various embodiments, the power supply 26a may comprise an electronic converter, such as a flyback, forward or half-bridge converter, comprising a transformer with a plurality of secondary windings, each secondary winding providing a respective supply voltage. Alternatively may be used a cascade of voltage regulators, wherein a first voltage regulator generates the supply voltage Vcc from the input voltage Vin and a second voltage regulator generates the voltage Vdd from the voltage Vcc. Generally, as mentioned before the power supply 26a may be also at least in part external with respect to the apparatus 20a. For example, an external AC/DC electronic converter may generate a DC voltage Vin from the mains, and one or more internal DC/DC voltage regulators may generate the voltages Vcc and Vdd. Generally, the mentioned DC/DC voltage regulators may be *e.g.* switched mode electronic converters *(e.g.* in order to reduce power losses) or linear regulators *(e.g.* in order to reduce the electromagnetic interference).

In various embodiments, the apparatus 20a may also comprise a user interface 50, *e.g.* for receiving an input from a user and/or for providing status information to the user. For example, the user interface 50 may be used to select a treatment program corresponding to one of the programs 1 to 9 (or a subset thereof) or a treatment application corresponding to a predetermined sequence of programs 1 to 9, *e.g.* programs 1, 3 and 6. Conversely, the status information shown to the user may include one or more of the following information: whether a treatment program is running, which treatment program has been selected, the time remaining of the treatment program, and/or diagnostic information, *e.g.* indicating that no antenna 30 has been connected to the terminals 202a and 202b. Accordingly, the user interface 50 may include buttons, a keyboard, luminous indicators (such as LEDs), a display, a touch screen, etc.

Figure 9 shows also an embodiment of the control circuit 22a. Specifically, in the embodiment considered, the control circuit 22a comprises a first digital processing circuit 220a, such as a microprocessor programmable via software instructions. Generally, the microprocessor may also form part of a microcontroller.

The first digital processing circuit 220a is connected to a non-volatile memory 222a. For example, the non-volatile 222a may correspond to the program memory of the microprocessor 220a, such as an EEPROM (Electrically Erasable Programmable Read-Only Memory) or a FLASH memory, *i.e.* the memory having stored the firmware/software executed by the microprocessor 220a. Specifically, in various embodiments, the non volatile memory 222a is used to store the timing data of the signal S to be generated, such as data identifying the frequency $f_{imp}$ of the base pulse I, optionally the type of the base pulse if different waveforms are supported, such as saw-tooth and profile with cusps, data identifying the durations $T_{pac\_on}$ and $T_{pac\_off}$ of a package P, and data identifying the durations $T_{tr\_on}$ and $T_{tr\_off}$ of a train Tr. As mentioned before, in various embodiments, the duration $T_{pac\_on}$ corresponds to a multiple of the period $T_{imp} = 1/f_{imp}$ and the duration $T_{tr\_on}$ corresponds to a multiple of the period $T_{pac} = T_{pac\_on} + T_{pac\_off}$.

In various embodiments, the control circuit 224 may also comprise a communication interface 224a for connection to an external processing system 10, such as a PC, a tablet, a smartphone or a remote server, *e.g.* a web-server. For example, the communication interface 224a may comprise at least one of:

- a reader device for a portable memory support;
- a communication interface for a wired communication with the processing system 10, such as a serial communication interface, *e.g.* a RS-232 or USB (Universal Serial Bus) communication interface, or an Ethernet network adapter;
- a short-range wireless communication interface, such as a NFC (Near Field Communciation) or Bluetooth® communication interface;
- a Wi-Fi communication interface according to the IEEE 802.11 standard; and
- a mobile communication interface, such as a GPRS (General Packet Radio Service), UMTS (Universal Mobile Telecommunications System) modem, HSPA (High-Speed Packet Access), or LTE (Long Term Evolution) modem.

Accordingly, the communication with the processing system 10 may be indirectly via a portable memory support, directly via a wired or wireless communication, via a LAN (Local-Area Network) or even via a WAN (Wide-Area Network), such as Internet. Generally, the communication interface and/or the memory 222a may also be integrated in a microcontroller comprising the microprocessor 220a. In the embodiment considered, the communication interface 224a may be used to communicate with the processing circuit 220a, *e.g.* in order to control the operation of the apparatus 20a. For example, the processing circuit 220a may be configured to implement one or more of the above functions described with respect to the user interface 50 by exchanging commands and/or status information with the processing system 10. For example, in this way, a smartphone or tablet may be used to control and/or monitor the operation of the apparatus 20a, *e.g.* via a Bluetooth® or Wi-Fi communication. In various embodiments, the processing circuit 220a may also be configured to receive new timing data of the signal S from the processing system 10 and store these data to the memory 222a. Similarly, in various embodiments, the communication interface 224a and the processing system 220a may also be configured to receive a new firmware for the processing unit 220a from the processing system 10 and store the new firmware to the memory 222a.

In various embodiments, the control circuit 22a comprises also a second digital processing circuit 220b, preferably a digital hardware circuit, such as an ASIC (Application-specific integrated circuit) or an FPGA (Field Programmable Gated Array). Accordingly, the control circuit 22a may also comprise a second non-volatile memory 222b having stored the firmware/program data for the second digital processing circuit 220b. In various embodiments, the communication interface 224a and the processing system 220a may thus also be configured to receive a new firmware for the processing circuit 220b from the processing system 10 and store the new firmware to the memory 222b.

Generally, the operation of the various circuits of the processing circuit 220b may also be implemented via software modules implementing the same function. For example, in this way, the operation of the processing circuit 220a and 220b may also be implemented in a single processing circuit, such as a DSP (Digital Signal Processor). However, as will be described in the following, several parallel processing operations are implemented within the processing circuit 220b, which would require a rather fast microprocessor in order to implement the same operations via software instructions. The inventor has observed that, also taking into account a typical production volume of the apparatus 20a, the most cost efficient solution is a microprocessor 220a configured to manage the user interface and the optional communication interface 224a, and a FPGA 220b for managing the generation of the signal S.

Specifically, in the embodiment considered, the second digital processing circuit 220b essentially implements a custom programmable signal generator, wherein the operation of the second digital processing circuit 220b is controlled by the first processing circuit 220a.

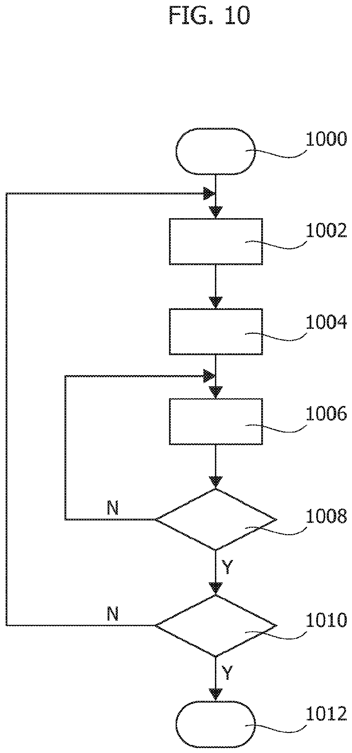

Figure 10 shows a flow chart of an embodiment of the operation of the first digital processing circuit 220a.

Specifically, in the flowchart, it is assumed that the first digital processing circuit 220a already has received at instructions (*e.g.* via the user interface 50 or the communication interface 224a) requesting the execution of a treatment application. As mentioned before, the treatment application may correspond to a given single treatment program selected, *e.g.* from one of the previously described programs 1 to 9, or a subset thereof, or a given sequence of treatment programs, *e.g.* programs 1, 3 and 6. Specifically, in various embodiments, the apparatus 20a may have stored the timing data of the previous described treatment programs 1 to 9. Conversely, in various embodiments, also different treatment programs may be stored. Specifically, the inventor has observed that the frequency $f_{imp}$ of the base pulse I (when maintained substantially constant) seems to be less relevant with the described shifting operation, while the most relevant data are the times $T_{pac}$ and $T_{tr}$ of the packets and trains, and the number $n_{pac}$. Accordingly, the programs could also have, *e.g.,* a different frequency $f_{imp}$. In various embodiments the frequency $f_{imp}$ is however still selected in a range between 100 Hz and 1 kHz, preferably between 100 and 400 Hz, even more preferably between 150 and 250 Hz.

For example, in various embodiments, a slight modification of program 5 has been performed:

- new program 5: saw-tooth base pulse with frequency $f_{imp}$ = 212.76 Hz, where the duration of the base pulse is $T_{imp}$ = 4.70 ms, the time $T_{pac\_on}$ is set to 75.20 ms (corresponding to the time of 16 base pulses), the pause between the packets is reduced to $T_{pac\_off}$ = 21 ms, the time $T_{tr\_on}$ is set to 384.80 ms (corresponding to the time of 4 packets) and the pause between the trains is $T_{tr\_off}$ = 370 ms;

Conversely, program 9 of document WO 2012/172504 A1 uses a base pulse with cusps and provides the broadest frequency spectrum with the highest number of harmonics. Generating such a profile with cusps may be rather complex with a digital circuit. Accordingly, in various embodiments, a similar broad frequency spectrum has been obtained by using the following timing data for a modified program 9:

- new program 9: saw-tooth base pulse with frequency $f_{imp}$ = 189.75 Hz, where the duration of the base pulse is $T_{imp}$ = 5.27 ms, the time $T_{pac\_on}$ is set to 26.35 ms (corresponding to the time of 5 base pulses), the pause between the packets is set to $T_{pac\_off}$ = 16.85 ms, the time $T_{tr\_on}$ is set to 1728.00 ms (corresponding to the time of 40 packets) and the pause between the trains is $T_{tr\_off}$ = 1600 ms.

Thus, in various embodiments, the other programs 1-4 and 6-8 may have the following characteristics:

- program 1: saw-tooth base pulse with frequency $f_{imp}$ = 212.76 Hz, where the duration of the base pulse is $T_{imp}$ = 4.70 ms, the time $T_{pac\_on}$ is set to 206.80 ms (corresponding to the time of 44 base pulses), the pause between the packets is $T_{pac\_off}$ = 140.00 ms, the time $T_{tr\_on}$ is set to 1387.20 ms (corresponding to the time of 4 packets) and the pause between the trains is $T_{tr\_off}$ = 1450 ms;
- program 2: saw-tooth base pulse with frequency $f_{imp}$ = 231.48 Hz, where the duration of the base pulse is $T_{imp}$ = 4.32 ms, the time $T_{pac\_on}$ is set to 146.88 ms (corresponding to the time of 34 base pulses), the pause between the packets is $T_{pac\_off}$ = 105.00 ms, the time $T_{tr\_on}$ is set to 1007.52 ms (corresponding to the time of 4 packets) and the pause between the trains is $T_{tr\_off}$ = 1100 ms;
- program 3: saw-tooth base pulse with frequency $f_{imp}$ = 212.76 Hz, where the duration of the base pulse is $T_{imp}$ = 4.70 ms, the time $T_{pac\_on}$ is set to 94.00 ms (corresponding to the time of 20 base pulses), the pause between the packets is $T_{pac\_off}$ = 55.00 ms, the time $T_{tr\_on}$ is set to 596.00 ms (corresponding to the time of 4 packets) and the pause between the trains is $T_{tr\_off}$ = 600 ms;
- program 4: saw-tooth base pulse with frequency $f_{imp}$ = 231.48 Hz, where the duration of the base pulse is $T_{imp}$ = 4.32 ms, the time $T_{pac\_on}$ is set to 77.76 ms (corresponding to the time of 18 base pulses), the pause between the packets is $T_{pac\_off}$ = 37.00 ms, the time $T_{tr\_on}$ is set to 459.04 ms (corresponding to the time of 4 packets) and the pause between the trains is $T_{tr\_off}$ = 480 ms;
- program 6: saw-tooth base pulse with frequency $f_{imp}$ = 231.48 Hz, where the duration of the base pulse is $T_{imp}$ = 4.32 ms, the time $T_{pac\_on}$ is set to 60.48 ms (corresponding to the time of 14 base pulses), the pause between the packets is $T_{pac\_off}$ = 16.00 ms, the time $T_{tr\_on}$ is set to 305.92 ms (corresponding to the time of 4 packets) and the pause between the trains is $T_{tr\_off}$ = 350 ms;
- program 7: saw-tooth base pulse with frequency $f_{imp}$ = 212.76 Hz, where the duration of the base pulse is $T_{imp}$ = 4.70 ms, the time $T_{pac\_on}$ is set to 56.40 ms (corresponding to the time of 12 base pulses), the pause between the packets is $T_{pac\_off}$ = 6.60 ms, the time $T_{tr\_on}$ is set to 252.00 ms (corresponding to the time of 4 packets) and the pause between the trains is $T_{tr\_off}$ = 220 ms;
- program 8: saw-tooth base pulse with frequency $f_{imp}$ = 231.48 Hz, where the duration of the base pulse is $T_{imp}$ = 4.32 ms, the time $T_{pac\_on}$ is set to 43.20 ms (corresponding to the time of 10 base pulses), the pause between the packets is $T_{pac\_off}$ = 2.44 ms, the time $T_{tr\_on}$ is set to 183.00 ms (corresponding to the time of 4 packets) and the pause between the trains is $T_{tr\_off}$ = 176 ms.

For example, the timing in [ms] of the above programs may be summarized according to the following table:

| # | $T_{imp}$ | $n_{imp}$ | $T_{pac\_on}$ | $T_{pac\_off}$ | $T_{pac}$ | $n_{pac}$ | $T_{tr\_on}$ | $T_{tr\_off}$ | $T_{tr}$ |
|---|---|---|---|---|---|---|---|---|---|
| 1 | 4.70 | 44 | 206.80 | 140.00 | 346.80 | 4 | 1387.20 | 1450 | 2837.20 |
| 2 | 4.32 | 34 | 146.88 | 105.00 | 251.88 | 4 | 1007.52 | 1100 | 2107.52 |
| 3 | 4.70 | 20 | 94.00 | 55.00 | 149.00 | 4 | 596.00 | 600 | 1196.00 |
| 4 | 4.32 | 18 | 77.76 | 37.00 | 114.76 | 4 | 459.04 | 480 | 939.04 |
| 5 | 4.70 | 16 | 75.20 | 21.00 | 96.20 | 4 | 384.80 | 370 | 754.80 |
| 6 | 4.32 | 14 | 60.48 | 16.00 | 76.48 | 4 | 305.92 | 350 | 655.92 |
| 7 | 4.70 | 12 | 56.40 | 6.60 | 63.00 | 4 | 252.00 | 220 | 472.00 |
| 8 | 4.32 | 10 | 43.20 | 2.55 | 45.75 | 4 | 183.00 | 176 | 359.00 |
| 9 | 5.27 | 5 | 26.35 | 16.85 | 43.20 | 40 | 1728.00 | 1600 | 3328.00 |

As described in the foregoing, in various embodiments, a PWM modulation may also be applied to each base pulse. For example, in various embodiments, a PWM modulation with a duty cycle of 50% is used, *i.e.* $T_{imp\_on} = T_{imp\_off} = T_{imp}/2$.

As mentioned before, the apparatus 20a may support only a single treatment application or support plural treatment applications. For example, in various embodiments, the apparatus 20a is configured to support at least the following treatment applications:

- a first treatment application executing only program 1;
- a second treatment application executing only program 3;
- a third treatment application executing only program 6;
- a fourth treatment application executing the sequence of programs 1, 3 and 6, wherein any order may be used, but preferably the order 1, 3 and 6 is used.

Specifically, in various embodiments, the apparatus 20a is configured to support a total and/or a local operating mode. Specifically, in the total operating mode, a large planar antenna 30 is connected to the apparatus 20a, thereby permitting a global/total stimulation of the body of the target (human). For example, in this case, the antenna 30 may be a planar pad or mat having a length between 120 cm and 250 cm, preferably between 150 cm and 200 cm, and a width between 40 cm and 120 cm, preferably between 60 cm and 90 cm. Conversely, in the local operating mode, a smaller antenna 30 is connected to the apparatus 20a, thereby permitting a local stimulation of only a part of the body of the target (human). For example, in this case, the antenna 30 may be a planar pad or mat having a length between 20 cm and 120 cm, preferably, between 20 cm and 60 cm, and a width between 20 cm and 60 cm, or an anatomically modeled antenna, *e.g.* having a shape being complementary to the area to be treated. Generally, the apparatus 20a may support only a single operating mode (total or local), or both operating modes. In the latter case, the apparatus 20a may comprise (in line with the description of Figure 2) two separate outputs: a first output configured to be connected to a global antenna and a second output configured to be connected to a local antenna. For example, in this case, the outputs may have different mechanical connectors. However, the apparatus 20a may also comprise only a single output and selection between the global or local operating mode may be performed explicitly via a switch of the user interface 50, or by selecting a given treatment application. For example, in various embodiments, the apparatus is configured to, once having selected a treatment application, execute first a respective total treatment application and then one or more respective local treatment applications. However, the apparatus may also be configured to permit a separate selection of the total and local treatment applications.

For example, in various embodiments, the apparatus 20a is configured to support one or more of the following global and/or local treatment applications:

- *e.g.* for treating osteoarticular and musculoskeletal pathologies, a total and/or a local (trauma area) treatment application executing both the sequence of programs 6 and 8;
- *e.g.* for treating vascular pathologies, a total and/or a local (trauma area) treatment application executing both the sequence of programs 6 and 5;
- *e.g.* for treating ulcers or a ischemic diabetic foot, a total and/or a local (ulcer area or foot) treatment application executing both the sequence of programs 6 and 8; and

- *e.g.* for treating a neuropathic diabetic foot, a total treatment application executing the sequence of programs 3, 6 and 8 and/or a local (foot) treatment application executing the sequence of programs 6 and 8.

As mentioned before, while also showing improvements for the programs 6 to 8 (and permitting a simplification of the implementation of the program 9), the apparatus 20a has been developed mainly in order to improve the programs 1 to 4. Accordingly, in addition to or as alternative to the above treatment applications, in various embodiments, the apparatus 20a is configured to support one or more of the following global and/or a local treatment applications:

- *e.g.* for treating multiple sclerosis, a total treatment application executing the sequence of programs 1, 3 and 6 and/or a local (head) treatment application executing the sequence of programs 3 and 6;
- *e.g.* for treating Parkinson's disease, a total treatment application executing the sequence of programs 3, 6 and 3 and/or a first local (head) treatment application executing the sequence of programs 3 and 6 and/or a second local (abdomen) treatment application executing the sequence of programs 8 and 8;
- *e.g.* for treating Alzheimer's disease or senile dementia, a total treatment application executing the sequence of programs 3, 6 and 9 and/or a local (head) treatment application executing the sequence of programs 3 and 6;
- *e.g.* for treating postictal states, a total treatment application executing the sequence of programs 3, 3 and 6 and/or a local (head) treatment application executing the sequence of programs 6 and 6;
- *e.g.* for treating fibromyalgia or chronic fatigue syndrome, a total treatment application executing the sequence of programs 3, 6 and 9 and/or a local (pain area) treatment application executing the sequence of programs 6 and 8; and
- *e.g.* for treating the Psycho-Neuro-Endocrine-Immunology (P.N.E.I.) system, a total treatment application executing the sequence of programs 9, 6 and 9 and/or a local (head) treatment application executing the sequence of programs 3 and 6.

Accordingly, usually a sequence of treatment programs has to be executed, wherein each treatment program may has associated respective timing data. Specifically, after a start step 1000, the first digital processing circuit 220a determines at a step 1002 the treatment program to be executed. As mentioned before, the treatment program may also correspond to the first treatment program of a treatment application. For example, the sequence of treatment program to be executed for a given treatment application may be stored in a Look-Up Table. In various embodiments, the first digital processing circuit 220a sends at the step 1002 one or more control commands CTRL to the second digital processing circuit 220b including data identifying the treatment program to be executed. For example, in various embodiment, the data identifying the treatment program to be executed may comprise timing data of the signal S. Accordingly, in this case, the first digital processing circuit 220a may read the timing data from the memory 222a and send at the step 1002 one or more control commands CTRL to the second digital processing circuit 220b, wherein the one or more control commands CTRL comprise the timing data of the signal S to be generated, such as:

- data identifying the frequency $f_{imp}$ of the base pulse I;
- optionally data identifying the duty cycle of the base pulse I;
- optionally the type of the base pulse if different waveforms are supported, such as saw-tooth and profile with cusps;
- data identifying the durations $T_{pac\_on}$ and $T_{pac\_off}$ of a package P;
- data identifying the durations $T_{tr\_on}$ and $T_{tr\_off}$ of a train Tr;
- optionally data identifying the inversion period of the signal S.

Generally, these data may correspond to the data stored to the memory 222a or may be determined as a function of the timing data stored to the memory 222a. Additionally or alternatively, the timing data of one or more treatment programs may also be stored directly in the second digital processing circuit 220b or in the memory 222b and read by the second digital processing circuit 220b. For example, in this way, a first set of standard programs, *e.g.* programs 1, 3 and 6, may already be pre-configured within the second digital processing circuit 220b, and one or more additional programs may be configured by storing the respective timing data to the memory 222a. Accordingly, in this case, the first digital processing circuit 220a may send at the step 1002 one or more control commands CTRL to the second digital processing circuit 220b, wherein the one or more control commands comprise a program number to be executed. Generally, the program number does not necessarily corresponds to the above mentioned treatment program numbers, but *e.g.* program 1 could correspond to treatment program 1, program 2 could correspond to treatment program 3, program 3 could correspond to treatment program 6.

At a step 1004, the first digital processing circuit 220a sends a control command CTRL to the second digital processing circuit 220b requesting that the generation of the signal S should be started with the characteristics communicated at the step 1002. Generally, the steps 1002 and 1004 may also be combined, because the data identifying the treatment program to be executed (step 1002) may also be included in the instruction requesting the generation of the signal S. Accordingly, in response to the start instruction, the second digital processing circuit 220b generates the signal S as will be described in greater detail in the following. In various embodiments, once having sent the start command (step 1004), the first digital processing circuit 220a proceeds then to a step 1006. The step 1006, essentially corresponds to a wait step. For example, during the step 1006, the first digital processing circuit 220a may monitor the operation of the second digital processing circuit 220b, *e.g.* by sending one or more control commands CTRL to the second digital processing circuit 220b requesting status data, or the second digital processing circuit 220b may send autonomously status data to the first digital processing circuit 220a.

In the embodiment considered, the first digital processing circuit 220a then verifies at a step 1008 whether the treatment time of the current treatment program has elapsed. For example, in various embodiments, the first digital processing circuit 220a monitors the treatment time and determines whether a predetermined treatment time associated with the current treatment program has been reached, such as 480 s. Additionally or alternatively, the second digital processing circuit 220b may monitor the treatment time and determine whether a predetermined treatment time associated with the current treatment program has been reached. In this case, the second digital processing circuit 220b may include in the status information (sent at the step 1006 to the first digital processing circuit 220a) data indicating whether the treatment program is running or whether the treatment time has elapsed. In case the first digital processing circuit 220a determines that the treatment time of the current treatment program has not elapsed (output "N" of the verification step 1008), the first digital processing circuit 220a returns to the step 1006. Conversely, in case the first digital processing circuit 220a determines that the treatment time of the current treatment program has elapsed (output "Y" of the verification step 1008), the first digital processing circuit 220a proceeds to a step 1010.

Specifically, in various embodiments, the first digital processing circuit 220a verifies at a step 1010 whether the current treatment program was the last treatment program to be executed. As mentioned before, a single treatment program or a sequence of treatment programs may be executed. In case the first digital processing circuit 220a determines that the current treatment program was not the last treatment program to be executed (output "N" of the verification step 1010), the first digital processing circuit 220a returns to the step 1002, where the next treatment program of a sequence of treatment programs is selected and the above procedure is repeated for the next treatment program. Conversely, in case the first digital processing circuit 220a determines that the current treatment program was the last treatment program to be executed (output "Y" of the verification step 1010), *e.g.* because only a single program has to be executed or because the treatment program was the last of a sequence of treatment programs, the procedure terminates at a stop step 1012. Generally, as described in the foregoing, the digital processing circuit 220a may also provide status data of the execution of the treatment application to the processing system 10.

Accordingly, in various embodiments, the second digital processing circuit 220b may be configured to support at least one of:

- a generic operation mode, in which the second digital processing circuit 220b is configured to generate the signal S as a function of timing data received from the digital processing circuit 220a; and/or
- one or more specific operation modes, in which the second digital processing circuit 220b is configured to generate the signal S as a function of timing data determined as a function of a program number received from the digital processing circuit 220a.

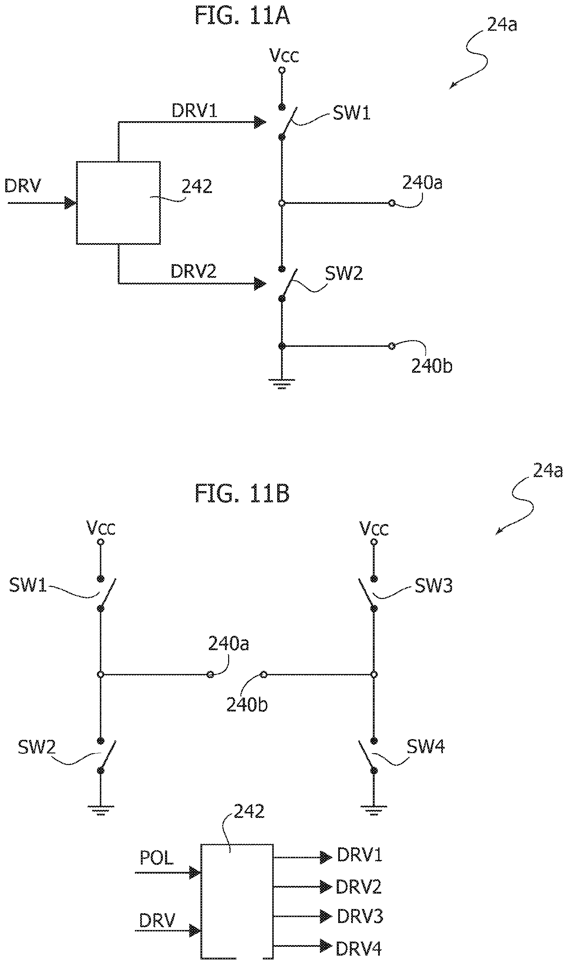

As described in the foregoing (see also Figure 9), in various embodiments, the power amplifier is implemented with a power/switching stage 24a and a filter stage 28, thereby forming a class D amplifier. For example, Figures 11A and 11B show possible embodiments of the power stage 24a. Specifically, the power stage 24a of Figure 11A is based on a half-bridge, and the power stage 24a of Figure 11B is based on a full-bridge.

For example, in Figure 11A, the power stage 24a comprises two electronic switches SW1 and SW2, such as Field-Effect Transistors (FET), *e.g.* MOSFETs, connected *(e.g.* directly) in series between the voltage Vcc and ground, wherein the intermediate node between the electronic switches SW1 and SW2 represents a switching node. Accordingly, the switching node may be set to Vcc (SW1 closed and SW2 opened) or ground (SW1 opened and SW2 closed). Accordingly, the output terminal 240a may correspond to the switching node between the switches SW1 and SW2, and the output terminal 240b may correspond to ground.

In the embodiment considered, the power stage 24a comprises thus also a driver circuit 242 configured to generate drive signals DRV1 and DRV2 for the electronic switches SW1 and SW2, respectively, wherein the drive signals DRV1 and DRV2 are generated as a function of the drive signal DRV. Generally, in such an arrangement, only one of the electronic switches of the half bridge is closed. For example, in various embodiments, the logic level of the drive signal DRV1 may correspond to the logic level of the drive signal DRV, and the logic level of the drive signal DRV2 may correspond to the inverted version of the logic level of the drive signal DRV.

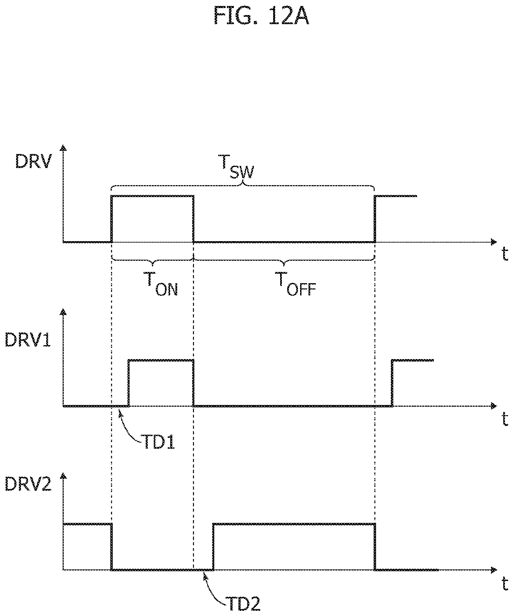

Conversely, Figure 12A shows an embodiment of the drive signals, wherein also dead times TD1 and TD2 are introduced between the edges of the drive signals. Generally, the PWM drive signal DRV is a pulsed signal, wherein the signal DRV is set to high for a given switch-on time Torr and to low for a given switch-off time $T_{OFF}$, wherein the switching period Tsw of the signal DRV corresponds to the sum of the switch-on time Torr and the switch-off time $T_{OFF}$

(Tsw = Torr + $T_{OFF}$), and the duty cycle D corresponds to the ratio between the switch-on time Torr and the switching period Tsw (D = $T_{ON}/T_{SW}$). Generally, while being preferable, it is not particularly relevant whether the switching period Tsw is constant, but the duty cycle D of the drive signal DRV should indicate a requested average voltage between the terminals 240a and 240b. For example, with such drive signal DRV, the driver circuit 242 may be configured to determine rising and falling edges in the signal DRV, and:

- in response to detecting a rising edge, set the signal DRV2 immediately to low and set the signal DRV1 to high after a delay TD1 (with respect to the rising edge); and
- in response to detecting a falling edge, set the signal DRV1 immediately to low and set the signal DRV2 to high after a delay TD2 (with respect to the falling edge).

Such delays may be useful in order to avoid that the switches of a half-bridge are closed contemporaneously. However, such delays TD1 and TD2 are usually small and thus will be neglected in the following.

Conversely, in Figure 11B, the power stage 24a comprises a first half-bridge comprising two electronic switches SW1 and SW2, such as FETs, *e.g.* MOSFETs, connected *(e.g.* directly) in series between the voltage Vcc and ground, and a second half-bridge comprising two electronic switches SW3 and SW4, such as FETs, *e.g.* MOSFETs, connected *(e.g.* directly) in series between the voltage Vcc and ground. Accordingly, the switching node between the electronic switches SW1 and SW2 and the switching node between the electronic switches SW3 and SW4 may be set to Vcc (SW1/SW3 closed and SW2/SW4 opened) or ground (SW1/SW3 opened and SW2/SW4 closed). In this case, the output terminal 240a may correspond to the switching node between the switches SW1 and SW2, and the output terminal 240b may correspond to the switching node between the switches SW3 and SW4. Specifically:

- when the switches SW1 and SW4 are closed (with the switches SW2 and SW3 opened), the voltage between the terminals 240a and 240b corresponds to Vcc,
- when the switches SW2 and SW3 are closed (with the switches SW1 and SW4 opened), the voltage between the terminals 240a and 240b corresponds to -Vcc,
- when the switches SW1 and SW3 are closed (with the switches SW2 and SW4 opened), the voltage between the terminals 240a and 240b corresponds to zero, and
- when the switches SW2 and SW4 are closed (with the switches SW1 and SW3 opened), the voltage between the terminals 240a and 240b corresponds to zero.

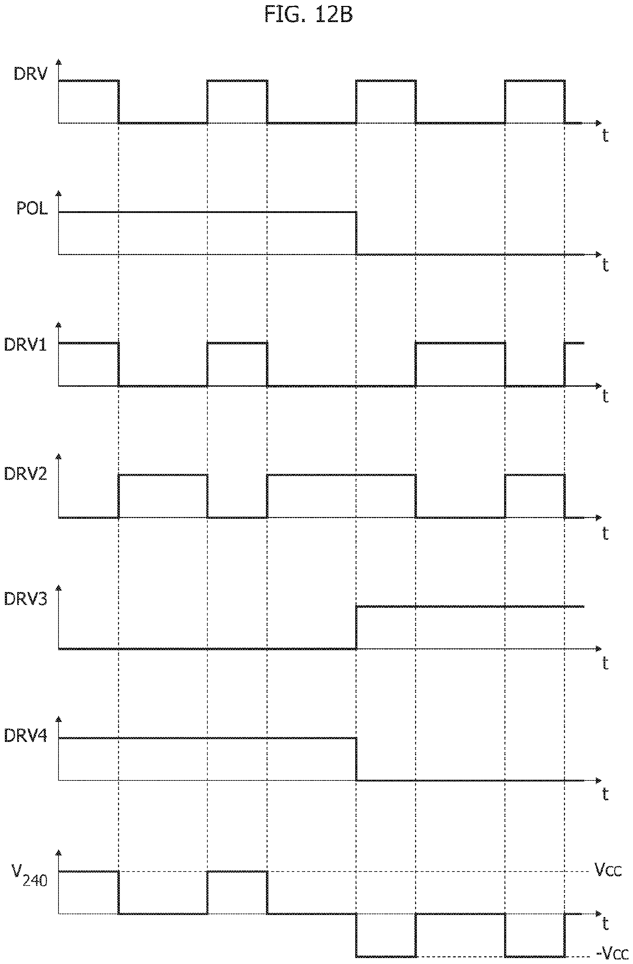

Accordingly, the arrangement of Figure 11B may be used when also the polarity of the signal S should be inverted (see Figure 6). In the embodiment considered, the power stage 24a comprises thus also a driver circuit 242 configured to generate drive signals DRV1, DRV2, DRV3 and DRV4 for the electronic switches SW1, SW2, SW3 and SW4, respectively, wherein the drive signals DRV1, DRV2, DRV3 and DRV4 are generated as a function of the drive signal DRV and a polarity signal POL. For example, Figure 12B shows an embodiment for generating the drive signals DRV1 to DRV4. Generally, Figure 12B does not show the dead times, but also in this case may be introduced dead times between the edges of the drive signals DRV1 and DRV, and the drive signals DRV3 and DRV4, respectively.

Specifically, in the embodiment considered, the driver circuit 242 is configured to determine rising and falling edges in the polarity signal POL and the drive signal DRV, and:

- in response to detecting a first type of edge *(e.g.* a rising edge) in the polarity signal POL, set the drive signal DRV3 to low (immediately) and the drive signal DRV4 to high (immediately or possibly after a delay), and:

  - in response to detecting a rising edge in the signal DRV, set the signal DRV1 to high (immediately or possibly after a delay) and set the signal DRV2 to low (immediately), whereby the voltage $V_{240}$ between the terminals 240a and 240b corresponds to Vcc, and
  - in response to detecting a falling edge in the signal DRV, set the signal DRV1 to low (immediately) and set the signal DRV2 to high (immediately or possibly after a delay), whereby the voltage $V_{240}$ between the terminals 240a and 240b corresponds to zero; and

- in response to detecting a second type of edge *(e.g.* a falling edge) in the polarity signal POL, set the drive signal DRV3 to high (immediately or possibly after a delay) and the drive signal DRV4 to low (immediately), and:

  - in response to detecting a rising edge in the signal DRV, set the signal DRV2 to high (immediately or possibly after a delay) and set the signal DRV1 to low (immediately), whereby the voltage $V_{240}$ between the terminals 240a and 240b corresponds to -Vcc, and
  - in response to detecting a falling edge in the signal DRV, set the signal DRV2 to low (immediately) and set the signal DRV1 to high (immediately or possibly after a delay), whereby the voltage $V_{240}$ between the terminals 240a and 240b corresponds to zero.

Accordingly, in various embodiments, the second digital processing circuit 220b is configured to generate the drive signal DRV for the power stage 24a, wherein the drive signal DRV corresponds to a PWM signal, and optionally the polarity signal POL. Generally, the information of the polarity signal POL may also be transmitted via the drive signal DRV, *e.g.* by switching the drive signal DRV between three levels ("+1", "0", "-1").

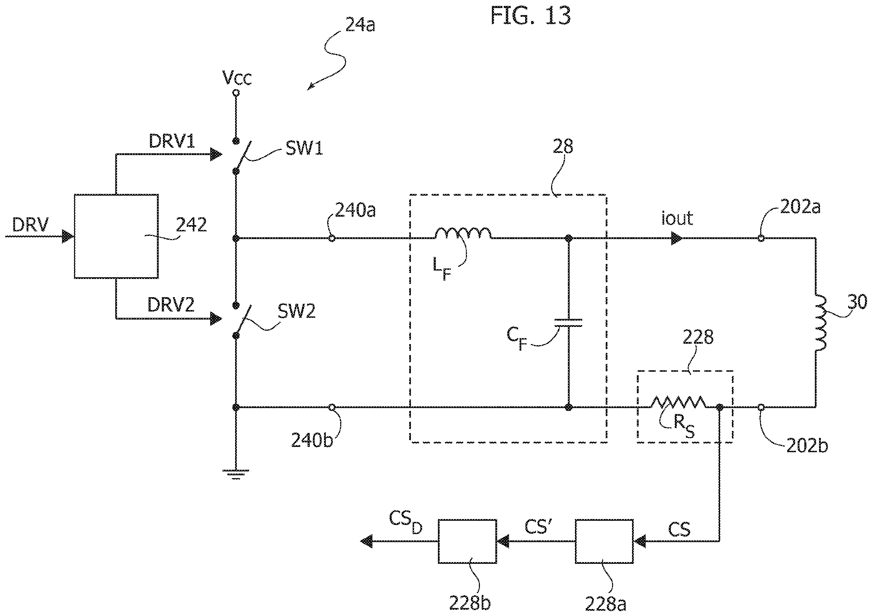

For example, Figure 13 shows an embodiment of the complete power amplifier at the example of a half-bridge as shown in Figure 11A, but also the full-bridge of Figure 11B could be used. Specifically, in the embodiment considered, the input terminals of the filter stage 28 are connected to the output terminals 240a and 240b of the power stage 24a, and the output terminals of the filter stage 28 are connected to the antenna 30, *e.g.* via the terminals 202a and 202b. For example, in the embodiment considered, the filter stage 28 implements an LC low pass filter. For example, in this case, and inductance $L_F$, such as an inductor may be connected between the terminals 240a and 202a, and a capacitance $C_F$, such as a capacitor may be connected between the terminals 202a and 202b. However, also other analog low-pass or band-pass filters may be used, preferably passive filters comprising only reactive components (inductances and capacitances). As mentioned before, the filter stage 28 may also comprise the inductance (and similarly capacitance and/or resistance) of the antenna 30.

Accordingly, the voltage applied to the antenna 30 (approximately) corresponds to the average voltage between the terminals 240a and 240b (*e.g.,* between Vcc and ground, or between Vcc and -Vcc). However, indeed the signal S should correspond to the current $i_{out}$ provided via the terminals 202a and 202b to the antenna 30. For this reason, in various embodiments, the apparatus 20a comprises a current sensor 228 configured to generate a signal CS indicative of (and preferably proportional to) the current $i_{out}$. For example, in various embodiments, the current sensor 228 is connected (*e.g.* directly) in series with the terminals 202a and 202b. However, the current sensor 228 may also be connected (*e.g.* directly) in series with the output terminals 240a and 240b of the power stage 24a, because the current provided by the power/switching stage 24a may also be used to estimate the current $i_{out}$. For example, the current sensor 228 may be a shunt resistor $R_S$, *e.g.* a shunt resistor $R_S$ connected in series with the terminals 202a and 202b, wherein the voltage at the resistor Rs is proportional to the current $i_{out}$ provided via the terminals 202a and 202b.

In this case, the second digital processing circuit 220b may be configured to vary the PWM drive signal DRV as a function of the signal CS in order to regulate the requested profile of the current $i_{out}$, *i.e.* of the signal S. Specifically, for this purpose, the control circuit 22a may comprise an analog-to-digital converter (A/D) 228b, such as a sigma-delta converter, which is configured to provide digital samples $CS_D$ of the signal CS.

Generally, *e.g.* in case the signal CS is not directly proportional to the current flowing through the antenna 30 (*e.g.* because the sensor 228 is connected to the output terminals of the power stage 24a), a low-pass filter 228a may be connected between the current sensor 228 and the A/D 228b, *i.e.* the A/D 228b may receive a low-pass filtered version CS' of the signal CS. Additionally or alternatively, the block 228a may comprise a rectifier circuit, *i.e.* the A/D 228b may receive a rectified version CS' of the signal CS. For example, this may be useful in case the polarity of the voltage between the terminals 240a and 240b may be inverted (see the description of Figures 11B and 12B). Generally, the low pass filtering and/or rectification of the block 228a may also be implemented digitally within the second digital processing circuit 220b.

Accordingly, in various embodiments, the second digital processing circuit 220b is configured to obtain digital samples $CS_D$ indicative of (and preferably proportional to) the (*e.g.* absolute value of the) current flowing through the antenna 30. As mentioned before, the second digital processing circuit 220b may be configured to vary the PWM drive signal DRV as a function of the samples $CS_D$. However, the samples $CS_D$ may also be used for other purposes. For example, the first or the second digital processing circuit 220a/220b may use the samples in order to determine whether the antenna 30 is disconnected, *e.g.* because the values $CS_D$ are below a given minimum threshold, or damaged, *e.g.* because the values $CS_D$ are above a given maximum threshold.

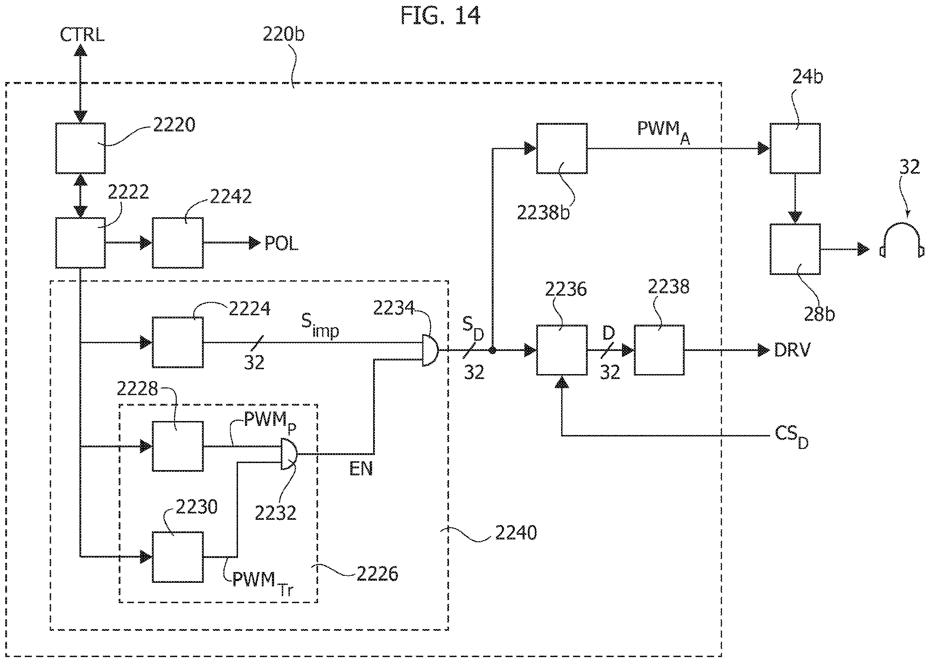

Figure 14 shows an embodiment of the second digital processing circuit 220b. Specifically, in the embodiment considered, the second digital processing circuit 220b is configured to generate the drive signal DRV as a function of digital data $S_D$ identifying a requested amplitude and the data $CS_D$ indicating the actual amplitude of the current flowing through the antenna 30. Substantially, in the embodiment considered, the data $S_D$ correspond to digital values of the signal S, *i.e.* the evolution of the values $S_D$ should correspond to the profile of the signal S described with respect to Figures 3 to 6 (with the additional shifting effect).

Accordingly, in the embodiment considered, the second digital processing circuit 220b comprises a digital hardware circuit 2240 configured to generate the sequence of values $S_D$ as a function of the timing data of the treatment program to be executed.

For example, in the embodiment considered, the second digital processing circuit 220b comprises a communication interface 2220 for exchanging data with the first digital processing unit 220a, in particular the previous mentioned control commands CTRL. Specifically, in various embodiments, the control commands CTRL comprise data identifying the treatment program to be executed, such as a program number or the respective timing data. For example, in various embodiments, the communication interface 2220 may be an Universal Asynchronous Receiver/Transmitter (UART), Inter-Integrated Circuit (I$^2$C) or Serial Peripheral Interface (SPI) communication interface.

In various embodiments, a circuit 2222 is thus configured to determine the timing data of the treatment program to be executed as a function of the data identifying the treatment program to be executed, *e.g.* by extracting the timing data from the control command(s) CTRL, or extracting the program number from the control command(s) CTRL and determining the timing data as a function of the program number. Generally, in the latter case, the timing data associated with a given the program number may be fixed or programmable, *e.g.* by storing respective timing data to the memory 222b.

In various embodiments, the circuit 2222 is configured to provide the following data to the digital hardware circuit 2240:

- data identifying the frequency $f_{imp}$ of the base pulse I;
- optionally data identifying the duty cycle of the base pulse I;
- optionally the type $P_{imp}$ of the base pulse if different waveforms are supported, such as saw-tooth and profile with cusps;
- data identifying the durations $T_{pac\_on}$ and $T_{pac\_off}$ of a package P;
- data identifying the durations $T_{tr\_on}$ and $T_{tr\_off}$ of a train Tr;
- optionally data identifying the inversion period of the signal S.

Figure 14 also shows a possible embodiment of the digital hardware circuit 2240. Specifically, in the embodiment considered, the digital hardware circuit 2240 comprises:

- a digital base pulse generator 2224 configured to generate a sequence of (continuous) digital samples $S_{imp}$ of the base pulse I as a function of the data identifying the frequency $f_{imp}$ of the base pulse I and optionally the type $P_{imp}$ of the base pulse; and
- an enable circuit 2226 configured to generate an enable signal EN as a function of the data identifying the durations $T_{pac\_on}$ and $T_{pac\_off}$ of a package P and data identifying the durations $T_{tr\_on}$ and $T_{tr\_off}$ of a train Tr.

For example, the digital samples $S_{imp}$ may have 8, 16, 24 or 32 bit.

In various embodiments, the digital hardware circuit 2240 comprises also logic gates 2234, such as a multiplexer or AND gates, configured to generate the values $S_D$ by:

- when the enable signal EN has a first logic level (*e.g.* high), setting the value $S_D$ to the value $S_{imp}$; and
- when the enable signal EN has a second logic level (*e.g.* low), setting the value $S_D$ to a predetermined value (*e.g.* zero) indicating that the output of the power stage 24a should be set to zero volt (see also the description of Figures 11 and 12.

Accordingly, in the embodiment considered, the signal $S_D$ (having usually the same number of bits as the signal $S_{imp}$) at the output of the logic gates 2234 corresponds to the signal S described with respect to Figure 8.

For example, in various embodiments, the enable circuit 2226 comprises:

- a first digital PWM generator circuit configured to generate a first PWM signal $PWM_P$, wherein, during each switching cycle with period $T_{pac}$, the signal $PWM_P$ is set to high for the time $T_{pac\_on}$ and low for the time $T_{pac\_off}$;
- a second digital PWM generator circuit configured to generate a second PWM signal $PWM_{Tr}$, wherein, during each switching cycle with period $T_{tr}$, the signal $PWM_{Tr}$ is set to high for the time $T_{tr\_on}$ and low for the time $T_{tr\_off}$; and
- a logic gate 2232, such as an AND gate, configured to generate the enable signal EN by combining the signals $PWM_P$ and $PWM_{Tr}$.

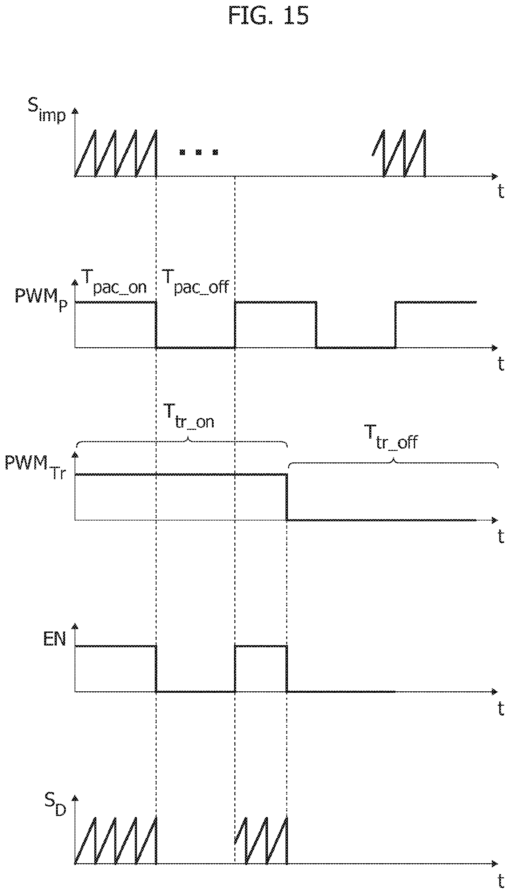

For example, this is also shown in Figure 15. Specifically, Figure 15 shows the variation of the digital values $S_{imp}$ which periodically follows the profile of the base pulse I, *e.g.* having a sawtooth profile. Moreover, the PWM signal $PWM_P$ is periodically set to high for the time $T_{pac\_on}$ and low for the time $T_{pac\_off}$, and the PWM signal $PWM_{Tr}$ is periodically set to high for the time $T_{tr\_on}$ and low for the time $T_{tr\_off}$, wherein the enable signal EN is set to high when the signals $PWM_P$ and $PWM_{Tr}$ are both high. Thus, as extremely shown in Figure 15, the pulses in the signal EN do not have the same duration because the frequency $f_{pac}$ is usually not a multiple of the frequency $f_{tr}$. Finally, the values $S_D$ are generated by modulating the base-pulse values $S_{imp}$ with the enable signal EN. However, also in this case, the signal $S_{imp}$ and the enable signal EN are not synchronized.

For example, the PWM signal generator circuits 2228 and 2230 may be implemented with one or more digital counters. For example, a PWM signal generator may be implemented by increasing a count value in response to a clock signal, wherein:

- the output of the PWM signal generator circuit is set to high when the count value is between 0 and a first value proportional to the time $T_{pac\_on}$ (or $T_{tr\_on}$);
- the output of the PWM signal generator circuit is set to low when the count value is greater than the first value; and
- the count value is reset to 0 when the count value reaches a second value proportional to the time $T_{pac}$ (or $T_{tr}$).

For example, the inventor has observed that with a clock frequency of 48 Mhz, the PWM signal generators 2228 and 2230 may be implemented with counters having 24 bits.

The base pulse generator circuit 2224 may have different implementation forms, which essentially depend on the fact whether the frequency $f_{imp}$ may be variable and whether a single or plural base pulse types are required. For example, in various embodiments, the second digital processing circuit 220b should be able to generate (at least) the programs 1, 3 and 6, *i.e.*:

- program 1: saw-tooth base pulse with frequency $f_{imp}$ = 212.76 Hz, where the duration of the base pulse is $T_{imp}$ = 4.70 ms, the time $T_{pac\_on}$ is set to 206.80 ms (corresponding to the time of 44 base pulses), the pause between the packets is $T_{pac\_off}$ = 140.00 ms, the time $T_{tr\_on}$ is set to 1387.20 ms (corresponding to the time of 4 packets) and the pause between the trains is $T_{tr\_off}$ = 1450 ms; and
- program 3: saw-tooth base pulse with frequency $f_{imp}$ = 212.76 Hz, where the duration of the base pulse is $T_{imp}$ = 4.70 ms, the time $T_{pac\_on}$ is set to 94.00 ms (corresponding to the time of 20 base pulses), the pause between the packets is $T_{pac\_off}$ = 55.00 ms, the time $T_{tr\_on}$ is set to 596.00 ms (corresponding to the time of 4 packets) and the pause between the trains is $T_{tr\_off}$ = 600 ms;
- program 6: saw-tooth base pulse with frequency $f_{imp}$ = 231.48 Hz, where the duration of the base pulse is $T_{imp}$ = 4.32 ms, the time $T_{pac\_on}$ is set to 60.48 ms (corresponding to the time of 14 base pulses), the pause between the packets is $T_{pac\_off}$ = 16.00 ms, the time $T_{tr\_on}$ is set to 305.92 ms (corresponding to the time of 4 packets) and the pause between the trains is $T_{tr\_off}$ = 350 ms;

Accordingly, these three programs use a saw-tooth base pulse, but two different frequencies $f_{imp}$ may be used. As mentioned before, also the modified program 9 may use a saw-tooth base pulse, but with a further frequency.

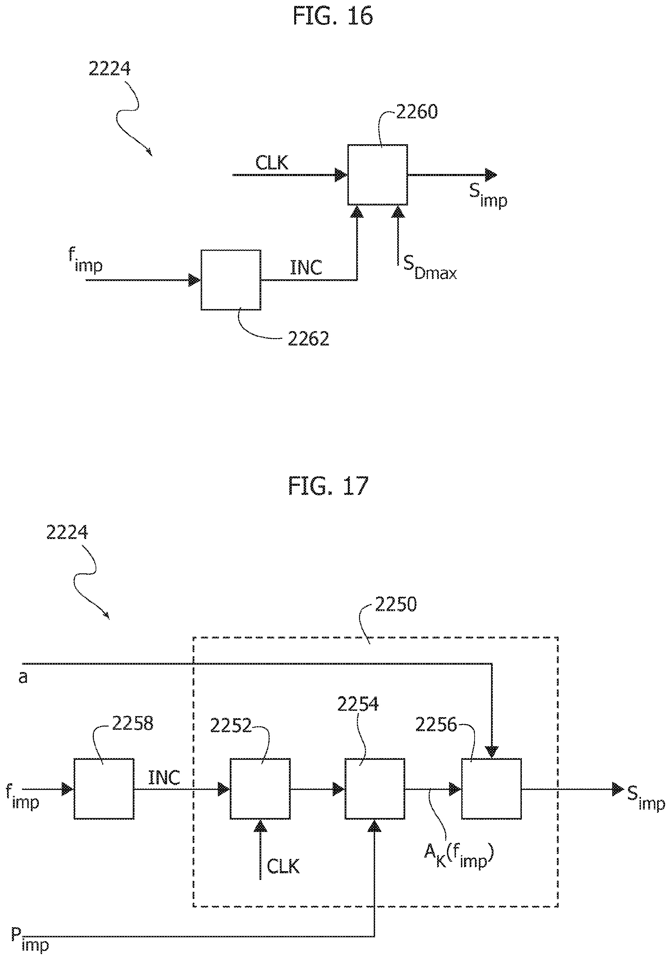

Figure 16 shows in this respect a possible embodiment of the base pulse generator circuit 2224 configured to generate saw-tooth base pulses with settable frequency. Specifically, in the embodiment considered, the base pulse generator circuit 2224 is implemented with a digital counter 2260 configured to:

- in response to a clock signal CLK having a given frequency $f_{CLK}$, increase a count value by a given increment value INC;
- reset the count value when the counter 2224 reaches a given maximum value $S_{Dmax}$.

Accordingly, a circuit 2262 may determine the number $n_{CLK}$ required to obtain a the requested frequency $f_{imp}$. For example, when receiving at input data indentifying the requested frequency, the circuit 2262 may calculate $n_{CLK}$ = $f_{CLK}/f_{imp}$. However, the circuit 2262 may also receive directly the number $n_{CLK}$, *i.e.* the data identifying the frequency $f_{imp}$ of the base pulse I may correspond to the number $n_{CLK}$. Next, the circuit 2262 may calculate the increment value INC as a function of maximum value $S_{Dmax}$ and the number $n_{CLK}$, *i.e.* INC = $S_{Dmax}/n_{CLK}$.

Generally, *e.g.* based on the resolution of the counter 2260, the calculated number $n_{CLK}$ and/or the increment value INC will not necessarily be an integer number. For example, assuming a clock frequency $f_{CLK}$ = 48 Mhz, and a requested frequency $f_{imp}$ = 212.76, $n_{CLK}$ would correspond to approximately 225,606.32 clock cycles. Moreover, assuming a counter with 24 bit, wherein the maximum value $SD_{max}$ is set to 16,777,215, the "optimal" increment value INC would be approximately 74.365. For example, when the counter uses the increment value INC = 74 indeed 226,720 clock cycles would be required to reach the maximum value $SD_{max}$, thereby resulting in a frequency of 211.715 Hz, and when the counter uses the increment value INC = 75 indeed 223,697 clock cycles would be required to reach the maximum value $SD_{max}$, thereby resulting in a frequency of 214.576 Hz.

Accordingly, in various embodiments, the circuit 2262 is configured to vary the increment value INC provided to the counter 2260, preferably for each clock cycle, such that the average value of the increment value INC corresponds the optimal increment value INC (with fraction). For example, for this purpose, the circuit 2262 may manage internally an increment value INC' having a resolution being greater than the resolution of the increment value INC provided to the counter 2260, wherein the value INC' is calculated according to the previous method by considering a given number of the most significant bits as integer part, wherein the given number corresponds to the number of bits of the signal INC, and the remaining least significant bits are considered as fraction part, *e.g.* the 16 least significant bits. Accordingly, the increment value INC may be varied such that the increment value INC corresponds in average to the value INC'.

In various embodiments, *e.g.* in order to permit a programmability of the base pulse profile or to support also other base pulse types, the base pulse generator circuit 2224 may also be based on a digital signal generator circuit using Direct Digital Synthesis (DDS). Figure 17 shows a possible embodiment of the base pulse generator circuit 2224 comprising a DDS signal generator 2250. In particular, DDS indicates a method for generating, using digital electronics, an arbitrary periodic waveform starting from a single reference oscillator. Basically, in this case, a look-up table (LUT) 2254 is used, wherein the LUT 2254 has stored the amplitudes $A_k$ for a given number of samples of a standard waveform with a frequency $f_s$. In various embodiments, the LUT 2254 may also have stored a plurality of standard waveforms, such as a saw-tooth waveform, a square waveform and/or the waveform comprising cusps (Figure 3d). Accordingly, in this case, a type signal $P_{imp}$ (provided by the circuit 2222) may be used to select one of the standard waveforms. Instead, for effective generation of the base pulse values $S_{imp}$ at the desired frequency, a counter 2252 is used that represents a phase accumulator, which is incremented by a given increment value INC at each iteration, *i.e.* in response to a clock signal CLK, where the increment value INC is determined as a function of the ratio between the frequency $f_{imp}$ of the required base pulse I and the frequency $f_s$ of the standard waveform. For example, similar to Figure 16, a circuit 2258 may be used, which is configured to calculate the increment value INC as a function of the frequencies $f_{imp}$ and $f_s$, *e.g.* INC = $f_{imp}/f_s$. Consequently, to determine the amplitude $A_k(f_{imp})$ of a base pulse at the desired frequency $f_{imp}$ at a given instant k, it is sufficient to use the value of the respective counter 2220, *i.e.,* the phase, as address for the LUT 2254, or the portion of the LUT associated with the waveform profile selected via the signal $P_{imp}$. In various embodiments, the amplitude $A_k(f_{imp})$ may refer to a standard amplitude, and a multiplier circuit 2256 may be configured to determine the value $S_{imp}$ by multiplying the value $A_k(f_{imp})$ with a coefficient a. Generally, the multiplier 2256 is purely optional and the value $S_{imp}$ may correspond to the value $A_k(f_{imp})$. Such data identifying the coefficient a may be received from the circuit 2222 similar to the timing data. For example, such data identifying the coefficient a may be stored for the treatment programs or received via the first digital processing circuit 220a from the user interface 50 or the communication interface 226a.

Thus, also in this case the optimal increment value INC may not be an integer value. Accordingly, also in this case, the circuit 2258 may manage internally an increment value INC' having a resolution being greater than the resolution of the increment value INC provided to the counter 2260, wherein the value INC' is calculated according to the previous method by considering a given number of the most significant bits as integer part, wherein the given number corresponds to the number of bits of the signal INC, and the remaining least significant bits are considered as fraction part, *e.g.* the 16 least significant bits. Accordingly, the increment value INC may be varied, preferably for each clock cycle, such that the increment value INC corresponds in average to the value INC'. For example, assuming a clock frequency of $f_{CLK}$ = 48 Mhz and standard waveforms being stored for base pulse profiles having $f_s$ = 10 Hz, the LUT would comprise 4,800,000 samples. Generally, instead of storing all these samples, the circuit 2224 may operate with a down-scaled version of the clock signal CLK, *i.e.* the circuit 2224 may operate with a frequency $f'_{CLK}$, *e.g.* corresponding to $f_{CLK}/4$, $f_{CLK}/8$, $f_{CLK}/16$, $f_{CLK}/24$ or $f_{CLK}/32$, etc. For example, in various embodiments, the frequency $f'_{CLK}$ is between 100 kHz and 2 MHz. For example, in the following will be assumed that the frequency $f'_{CLK}$ corresponds to 375,000 Hz ($f_{CLK}/128$), *i.e.* for $f_s$ = 10 Hz, the LUT would comprise 37,500 samples. Accordingly, in order to generate a base pulse with $f_{imp}$ = 212.76, the internal increment value INC' may comprise the bits of the signal INC for the integer part and *e.g.* 10 bits for the fraction part. Accordingly the internal increment value INC' would correspond to 21.276, *e.g.* binary encoded as "10101.0100011010", which approximately corresponds to 21.27539. Thus, the circuit 2258 may set the increment value INC either to 21 ("10101") or 22 ("10110"), such that the average value of the increment value INC corresponds to the value INC'. For example, in the embodiment considered, the fractional part ".0100011010" corresponds to "0100011010" / "10000000000" = 282/1024. Accordingly, the circuit 2258 may be configured to apply 282 times the value 22 and 1024-282 = 742 times the value 21, which would result in an average increment value of (282 x 22 + 742 x 21)/1024 = 21.27539.

The inventor has observed that this averaging operation implemented by the circuits 2258 and 2262 may introduces a further small dithering operation of the harmonics of the base pulse I, which seems to be useful in order to improve the result of the low frequency treatment programs. Thus, in various embodiments, a first dithering operation may be performed directly during the generation of the base pulse values $S_{imp}$ by the averaging operation of the increment value INC provided to the signal generator 2250 or 2260. A second dithering operation (which indeed is not casual due to the shifting effect) is then performed by enabling and disabling the base pulse values $S_{imp}$ according to the timing of the packet P and the train Tr.

As describe in the foregoing, a PWM modulation may also be applied to the base pulse I. For example, in the embodiment shown in Figure 16, the counter 2260 may directly compare the count value with a further threshold (being smaller than SDmax), wherein the further threshold is indicative of the duty cycle. Accordingly, when the count value is greater than the further threshold, the counter 2260 may set the value $S_{imp}$ to zero. Similarly, also in the embodiment shown in Figure 17 the count/phase value provided by the counter 2252 may be compared with a threshold (being smaller than the number of sample stored to the LUT 2254), wherein the further threshold is indicative of the duty cycle. Accordingly, when the count value is greater than the further threshold, a combinational logic circuit (*e.g.* within the block 2256) may set the value $S_{imp}$ to zero. However, in Figure 17, the LUT 2254 may already have stored the profile of one or more standard waveforms having already applied a PWM modulation.

The inventor has observed that the disclosed digital solution provides surprisingly better treatment results than a complete analog implementation of the block 2240, or a mixed digital/analog solution comprising an analog waveform generator (implementing the operation of the block 2224) and a digital circuit (implementing the operation of the clock 226) configured to selectively connected the output of the analog waveform generator to a power amplifier. Presumably, this results from the fact that the described digital implementation of the circuit 2224 permits to control more precisely the frequency of the base pulses. In fact, the described solutions permit to obtain a precise clock frequency, being not correlated with the frequency of the packets and trains, which ensures that the harmonics do not overlap. Moreover, the additional dithering operation of the averaging operation permits to slightly broaden the harmonics of the base pulses.

A further possible reason may reside in the additional control of the current flowing through the antenna 30. Specifically, as described in the foregoing, the values $S_D$ represent the requested values of the current $i_{out}$ to be provided to the antenna 30. Specifically, as shown in Figure 14, in various embodiments, the value $S_D$ and the sample $CS_D$ indicative of the current provided to the antenna 30 are provided to a digital regulator circuit 2236 comprising an Integral (I) component and a Proportional (P) components. Specifically, the digital regulator circuit 2236 is configured to generate a digital signal D indicative of the duty cycle of the signal DRV as a function of the requested values $S_D$ and the actual value $CS_D$. Substantially, the regulator circuit 2236 is configured to vary (via the PI regulation) the digital value D such that the value $CS_D$ corresponds to the value $S_D$, *i.e.* increases the value D when the value $CS_D$ is smaller than the value $S_D$ and decreases the value D when the value $CS_D$ is greater than the value $S_D$. Digital/discrete implementations of PI (or PID) regulators are *per se* well known in the art, *e.g.* from the respective Wikipedia page (version of April 19, 2020) relating to "PID controller", in particular the section "Discrete implementation", or document AN21990-13, "ADSP-21990: Implementation of PI Controllers" Analog Devices Inc., December 2001. Generally, with such a PI (or PID) regulator, the value $S_D$ represents the set-point and the value $CS_D$ represents the process variable. For example, a PI regulator may perform the following operations for each step k (which may correspond to a single clock cycle or a plurality of clock cycles):

- calculate an error value E(k) as a function of the difference between the set-point $S_D(k)$ and the process variable $CS_D(k)$, *i.e.:*

$$E(k) = S_D(k) - CS_D(k)$$

- calculate, *e.g.* via an accumulator, an integral value EI(k) indicative of the integral of the error values E(k), *e.g.:*

$$EI(k) = EI(k\text{-}1) + E(k), \text{ with } EI(0) = 0;$$

- calculate the output D(k) as a function of the proportional component E(k) and the integral component EI(k) by using respective coefficients Kp and Ki, *i.e.:*

$$D = Kp \times E(k) + Ki \times EI(k)$$

In the embodiment considered, the value D is provided to a digital PWM generator circuit 2238 configured to generate the drive signal DRV as a function of the signal D. For example, also the PWM signal generator 2238 may be implemented with one or more digital counters. For example, the PWM signal generator 2238 may be implemented by increasing a count value in response to the clock signal CLK, wherein:

- the signal DRV at the output of the PWM signal generator 2238 is set to high when the count value is between 0 and a first value proportional to (and preferably corresponding to) the value D;
- the output of the PWM signal generator is set to low when the count value is greater than the first value; and
- the count value is reset to 0 when the count value reaches a given maximum value.

For example, in various embodiments, the counter 2238 has the same number of bits as the signal $S_D$, *e.g.* 8 bits, which *e.g.* permits to obtain a PWM signal DRV with a switching frequency of 48 MHz/256 = 187,500 Hz.

Thus, in various embodiments, the digital PI regulator 2236, the digital PWM signal generator 2238, the power/switching stage 24a, the filter 28, the current sensor 228 and the A/D 228b (and possibly the filter 228a) implement a regulated current generator, configured to regulate the output current $i_{out}$ provided via the terminals 202a and 202b to the requested value $S_D$. For example, the embodiment shown in Figure 13 essentially implements a buck converter.

However, the implementation of the digital PI regulator 2236 and the digital PWM signal generator 2238 within the second digital processing circuit 220b has several advantages compared to the use of a separate regulated current generator. For example, the solution is more cost efficient, because instead of using a digital-to analog (D/A) converter for the signal $S_D$ and a separate regulated current generator, only an A/D converter 228b is required and the control operation of the regulated current generator (usually implemented in a dedicated current control IC) may be implemented by digital processing within the second processing circuit 220b. Moreover, due to the implementation within a FPGA or ASIC, a faster regulation of the current $i_{out}$ is possible, thereby permitting that the current $i_{out}$ follows precisely the profile of the values $S_D$. In fact, such control ICs for regulated current generators usually are for constant current applications, such as for powering LEDs, and thus do not provide a sufficient bandwidth in order to follow a constantly varying profile of current pulses. Moreover, also the inversion of the polarity may be implemented easier. In fact, as described in the foregoing, the power/switching stage (while amplifying the PWM signal DRV) may also invert the polarity of the voltage between the terminals 240a and 240b as a function of a polarity signal POL.

For example, as shown in Figure 14, for this purpose, the second digital processing circuit 220b may comprise a further signal generator 2242 configured to generate the polarity signal POL as a function of timing data received from the circuit 2222. Substantially, in various embodiments, the polarity signal corresponds to a PWM signal with 50% duty cycle. For example, the signal generator circuit 2242 may be implemented with a counter, *e.g.* configured to increase a count value in response to a clock signal, wherein the output of the signal generator 2242 is inverted and the count value is reset to 0 when the count value reaches a threshold value proportional to the inversion period, *e.g.* 120 s.

Finally, as shown in Figure 14, in various embodiments the apparatus 20a may also be configured to generate one or more further signals to be applied to other transducers, such as acoustic, light or haptic transducers, in order to further stimulate the patient/target. For example, such a further signal may be generated by implementing a further class D amplifier comprising:

- a digital PWM signal generator 2236b configured to generate a PWM signal $PWM_A$, wherein the duty cycle of the signal $PWM_A$ is proportional to the signal $S_D$, wherein the digital PWM signal generator 2236b is preferably integrated in the second digital processing circuit 220b;
- a power/switching stage 24b configured to generate an amplified PWM signal by amplifying the PWM signal $PWM_A$; and
- an analog low-pass or band-pass filter 28b configured to generate the further signal by filtering the amplified PWM signal generated by the power/switching stage 24b, wherein the power/switching stage 24b and the filter 28b are preferably external with respect to the second digital processing circuit 220b.

For example, in this way may be generated one or more further signals, synchronized with the signal applied to the antenna 30. For example, the further signal may be at least one of:

- as shown in Figure 14, an audio signal, which *e.g.* may be applied to an audio jack, which in turn may be connected to a headphone or speaker 32 configured to receive such an audio signal;
- a signal used to drive one or more light sources, such as *e.g.* LEDs, configured to be arranged in the vicinity of the eyes of the patient, *e.g.* by mounting the light sources in a headset or glasses;
- a signal used to drive a vibration transducers, such as a piezoelectric vibration transducer, *e.g.* incorporated in a global and/or local antenna 30.

Accordingly, in various embodiments, the further signal(s) use the same signal profile $S_D$ (frequencies of base pulses, packets and trains) generated for the antenna(s) 30 (total and/or local applicators). The further signal(s) are supplied in a synchronous mode, *e.g.* to drive a headphone, LED glasses and piezoelectric Transducers. Accordingly, these one or more additional stimuli may be perceived by receptors of the patient, thereby stimulating given areas in the brain of the patient associated with the respective receptors, such as acoustic receptors (audio), visual receptors (light), and/or proprioceptive and/or nociceptive receptors (vibration).

Specifically, *e.g.* because the transducers are not fast enough or similarly because human eyes would be unable to perceive the variation of saw-tooth base pulses having a frequency around of 200 Hz, in various embodiments, one or more further signals may be generated by filtering the samples $S_D$. For example, in various embodiments, a binarized version of the samples $S_D$ is used. For example, as described in the foregoing, in various embodiments, the base pulses I uses a PWM modulation, *e.g.* of 50%. Accordingly, in this case, the digital PWM signal generator 2236b may be configured to generate the PWM signal $PWM_A$ by setting the signal $PWM_A$ to high when the value $S_D$ is greater than a first threshold, *e.g.* zero, and to low when the value $S_D$ is smaller or equal to the first threshold, *e.g.* zero. For example, this simplifies also the generation of further signal, because the low-pass filter 28b may be omitted. Moreover, for the above mentioned transducers, the value of the polarity signal POL is not relevant.

Accordingly, in various embodiments, one or more further signals (such as the audio signal and the signal used to drive one or more LEDs, and optionally the signal used to drive a vibration transducers) have a square waveform, but follow the timing of the base pulses ($T_{imp}$, $T_{imp\_on}$, $T_{pac}$, $T_{tr}$). For example, test have demonstrated that, while a human brain is typically able to elaborate images with up to 30 fps, indeed a human eye is able to perceive frequencies up to (approximately) 250 Hz.

Accordingly, various embodiments relate to a system for therapeutic treatments with electromagnetic waves. The system comprises an antenna 30 and an apparatus 20a configured to generate a supply current $i_{out}$ for the antenna 30 in order to generate the electromagnetic waves. In various embodiments, the apparatus 20a comprises a digital processing circuit 220b.

According to the first aspect, the digital processing circuit 220b is configured to generate a sequence of values $S_D$ corresponding to the signal S shown in Figures 8 and 15. Specifically, in various embodiments, in order to generate the sequence of values $S_D$, the digital processing circuit 220b is configured to generate via the circuit or module 2224 a sequence of digital values $S_{imp}$ of a periodic base pulse I having a given frequency $f_{imp}$. The digital processing circuit 220b is also configured to generate via the circuit or module 2228 a PWM signal $PWM_P$, wherein the PWM signal $PWM_P$ is set, for each switching cycle $T_{pac}$, to a respective first logic level (*e.g.* high) for a packet switch-on period $T_{pac\_on}$ and to a respective second logic level (*e.g.* low) for a packet switch-off period $T_{pac\_off}$, and via the circuit or module 2230 a PWM signal $PWM_{Tr}$, wherein the PWM signal $PWM_{Tr}$ is set, for each switching cycle $T_{tr}$, to a respective first logic level (*e.g.* high) for a train switch-on period $T_{tr\_on}$ and to a respective second logic level (*e.g.* low) for a train switch-off period $T_{tr\_off}$. Moreover, the digital processing circuit 220b is configured to generate via the circuit or module 2232 an enable signal EN, wherein the enable signal EN is set to a respective first logic level (*e.g.* high) when the PWM signal $PWM_P$ has the respective first logic level (*e.g.* high) and the PWM signal $PWM_{Tr}$ has the respective first logic level (*e.g.* high), and to a respective second logic level (*e.g.* low) when the PWM signal $PWM_P$ has the respective second logic level (*e.g.* low) or the PWM signal $PWM_{Tr}$ has the respective second logic level (*e.g.* low). Specifically, in this case, the digital processing circuit 220b is configured to generate via the circuit or module 2234 the digital value $S_D$, wherein the digital value $S_D$ is set to the digital value $S_{imp}$ when the enable signal EN has the respective first logic level (*e.g.* high) and to zero when the enable signal EN has the respective second logic level (*e.g.* low).

Generally, these values $S_D$ could be provided to the antenna via an A/D converter. Conversely, according to a second aspect, the digital processing circuit 220b is configured to generate a PWM signal DRV having a given duty cycle, wherein the PWM signal DRV is set, for each switching cycle Tsw, to high for a switch-on period Torr and to low for a switch-off period $T_{OFF}$. In this case, the apparatus 20a comprises also a switching stage 24a configured to generate an amplified PWM signal $V_{240}$ by amplifying the PWM signal DRV and an analog low-pass or band-pass filter 28 configured to generate the supply current $i_{out}$ by filtering the amplified PWM signal $V_{240}$.

Accordingly, in this case, the digital processing circuit 220b may be configured to generate the PWM signal DRV as a function of the digital value $S_D$. For example, in various embodiments, the apparatus comprises also a current sensor 228 configured to provide a digital sample $CS_D$ indicative of the amplitude of the supply current $i_{out}$. In this case, the digital processing circuit 220b may be configured to generate via the circuit or module 2238 the PWM signal DRV as a function of a digital value D indicative of a duty cycle of the PWM signal DRV. Moreover, the digital processing circuit 220b may be configured to vary the digital value D via a discrete proportional-integral regulation configured to regulate the difference between the digital value $S_D$ and the digital sample $CS_D$ to zero.

Of course, without prejudice to the principle of the invention, the details of construction and the embodiments may vary widely with respect to what has been described and illustrated herein purely by way of example, without thereby departing from the scope of the present invention, as defined by the ensuing claims.

**Claims**

1. A system for therapeutic treatments with electromagnetic waves, comprising an antenna (30) and an apparatus (20a) configured to generate a supply current ($i_{out}$) for said antenna (30) in order to generate said electromagnetic wave, wherein said apparatus (20a) comprises:

   - a digital processing circuit (220b) configured to generate a first PWM signal (DRV) having a given duty cycle, wherein said first PWM signal (DRV) is set, for each switching cycle (Tsw), to high for a switch-on period (Torr) and to low for a switch-off period ($T_{OFF}$);
   - a switching stage (24a) configured to generate an amplified PWM signal ($V_{240}$) by amplifying said first PWM signal (DRV);
   - an analog low-pass or band-pass filter (28) configured to generate said supply current ($i_{out}$) by filtering said amplified PWM signal ($V_{240}$);
   - a current sensor (228) configured to provide a digital sample ($CS_D$) indicative of the amplitude of said supply

22

current ($i_{out}$);

wherein said digital processing circuit (220b) is configured to:

- generate (2224) a sequence of first digital values ($S_{imp}$) of a periodic base pulse (I) having a given frequency ($f_{imp}$);
- generate (2228) a second PWM signal ($PWM_P$), wherein said second PWM signal ($PWM_P$) is set, for each switching cycle ($T_{pac}$), to a respective first logic level for a packet switch-on period ($T_{pac\_on}$) and to a respective second logic level for a packet switch-off period ($T_{pac\_off}$);
- generate (2230) a third PWM signal ($PWM_{Tr}$), wherein said third PWM signal ($PWM_{Tr}$) is set, for each switching cycle ($T_{tr}$), to a respective first logic level for a train switch-on period ($T_{tr\_on}$) and to a respective second logic level for a train switch-off period ($T_{tr\_off}$);
- generate (2232) an enable signal ($\overline{EN}$), wherein said enable signal (EN) is set to a respective first logic level when said second PWM signal ($PWM_P$) has the respective first logic level and said third PWM signal ($PWM_{Tr}$) has the respective first logic level, and to a respective second logic level when said second PWM signal ($PWM_P$) has the respective second logic level or said third PWM signal ($PWM_{Tr}$) has the respective second logic level;
- generate (2234) a second digital value ($S_D$), wherein the second digital value ($S_D$) is set to the first digital value ($S_{imp}$) when said enable signal (EN) has the respective first logic level and to zero when said enable signal (EN) has the respective second logic level;
- generate (2238) said first PWM signal (DRV) as a function of a third digital value (D) indicative of said given duty cycle;
- vary said third digital value (D) via a discrete proportional-integral regulation configured to regulate the difference between said second digital value ($S_D$) and said digital sample ($CS_D$) to zero.

2. The system for therapeutic treatments according to Claim 1, wherein said digital processing circuit (220b) is implemented with an FPGA or an ASIC comprising:

- a digital signal generator (2224) configured to generate said sequence of first digital values ($S_{imp}$);
- a first digital PWM generator circuit (2238) configured to generate said first PWM signal ($PWM_P$) as a function of said third digital value (D) indicative of said given duty cycle;
- a second digital PWM generator circuit (2228) configured to generate said second PWM signal ($PWM_P$);
- a third digital PWM generator circuit (2230) configured to generate said third PWM signal ($PWM_{Tr}$);
- a first logic gate (2232) configured to generate said enable signal (EN) as a function of said second PWM signal ($PWM_P$) and said third PWM signal ($PWM_{Tr}$);
- a second logic circuit (2234) configured to generate said second digital value ($S_D$) as a function of said first digital value ($S_{imp}$) and said enable signal (EN);
- a discrete proportional-integral regulator circuit (2236) configured to vary said third digital value (D) as a function of said second digital value ($S_D$) and said digital sample ($CS_D$).

3. The system for therapeutic treatments according to Claim 2, wherein said digital signal generator (2224) is configured to generate periodic base pulses (I) having a saw-tooth profile, and wherein said digital signal generator (2224) comprises a counter (2260) configured to:

- in response to a clock signal (CLK) having a given frequency ($f_{CLK}$), increase said first digital values ($S_{imp}$) by an increment value (INC); and
- reset said first digital values ($S_{imp}$) when said first digital values ($S_{imp}$) reaches a given maximum value ($S_{Dmax}$).

4. The system for therapeutic treatments according to Claim 3, wherein said digital signal generator (2224) comprises an increment control circuit (2262) configured to generate said increment value (INC) as a function of:

- data identifying said frequency ($F_{CLK}$) or said clock signal (CLK);
- data identifying said frequency ($f_{imp}$) of said periodic base pulses (I);
- said maximum value (Somax).

5. The system for therapeutic treatments according to Claim 2, wherein said digital signal generator (2224) comprises:

- a look-up table (2254) comprising a plurality of elements corresponding to a given number of samples of a standard waveform of said base pulses (I) with a given standard frequency (fs), wherein each element has

stored the amplitude ($A_k$) of a respective sample of said standard waveform of said base pulses (I), wherein said look-up table (2254) receives at input a count value selecting a given sample, and wherein said first digital value ($S_{imp}$) is determined as a function of the amplitude ($A_k$) of the respective selected sample;
- a counter (2252) configured to, in response to a clock signal (CLK), increase said count value by an increment value (INC), whereby said counter (2252) represents a phase accumulator; and
- an increment control circuit (2258) configured to generate said increment value (INC) as a function of data identifying said standard frequency (fs) and data identifying said frequency ($f_{imp}$) of said periodic base pulses (I).

6. The system for therapeutic treatments according to Claim 4 or Claim 5, wherein said increment control circuit (2262; 2258) is configured to calculate an internal increment value (INC'), said internal increment value (INC') having an integer part having a number of bits corresponding to the number of bits of said increment value (INC), and a fractional part, and wherein said increment control circuit (2262; 2258) is configured to vary said increment value (INC), such that said increment value (INC) has an average value corresponding to said internal increment value (INC').

7. The system for therapeutic treatments according to any of the previous claims, wherein said digital processing circuit (220b) comprises a third digital PWM generator circuit (2242) configured to generate a polarity signal (POL), and wherein said switching stage (24a) comprises:

    - a first half-bride comprising a first (SW1) and a second (SW2) electronic switch, wherein the intermediate node between said first (SW1) and said second (SW2) electronic switch is a first switching node;
    - a second half-bride comprising a third (SW3) and a fourth (SW4) electronic switch, wherein the intermediate node between said third (SW3) and said fourth (SW4) electronic switch is a second switching node, wherein said amplified PWM signal ($V_{240}$) corresponds to the voltage between said first switching node and said second switching node;
    - a driver circuit (242) configured to generate drive signals (DRV1-DRV4) for said first (SW1), second (SW2), third (SW3) and fourth (SW4) electronic switch as a function of said first PWM signal (DRV) and said polarity signal (POL).

8. The system for therapeutic treatments according to any of the previous claims, wherein said digital processing circuit (220b) comprises:

    - a first communication interface (2220) for exchanging data with a further digital processing unit (220a); and
    - a control circuit (2222) configured to receive via said first communication interface (2220) data identifying a treatment program to be executed and, as a function of said data identifying a treatment program to be executed determine:

        - said frequency ($f_{imp}$) of said periodic base pulse (I),
        - said packet switch-on period ($T_{pac\_on}$) and said packet switch-off period ($T_{pac\_off}$), and
        - said train switch-on period ($T_{tr\_on}$) and said train switch-off period ($T_{tr\_off}$).

9. The system for therapeutic treatments according to Claim 8, wherein said digital processing circuit (220b) and said further digital processing unit (220a) are configured to execute at least one of the following treatment programs:

    - program 1: saw-tooth base pulse with frequency $f_{imp}$ = 212.76 Hz, where the duration of the base pulse is $T_{imp}$ = 4.70 ms, the time $T_{pac\_on}$ is set to 206.80 ms (corresponding to the time of 44 base pulses), the pause between the packets is $T_{pac\_off}$ = 140.00 ms, the time $T_{tr\_on}$ is set to 1387.20 ms (corresponding to the time of 4 packets) and the pause between the trains is $T_{tr\_off}$ = 1450 ms;
    - program 2: saw-tooth base pulse with frequency $f_{imp}$ = 231.48 Hz, where the duration of the base pulse is $T_{imp}$ = 4.32 ms, the time $T_{pac\_on}$ is set to 146.88 ms (corresponding to the time of 34 base pulses), the pause between the packets is $T_{pac\ off}$ = 105.00 ms, the time $T_{tr\_on}$ is set to 1007.52 ms (corresponding to the time of 4 packets) and the pause between the trains is $T_{tr\_off}$ = 1100 ms;
    - program 3: saw-tooth base pulse with frequency $f_{imp}$ = 212.76 Hz, where the duration of the base pulse is $T_{imp}$ = 4.70 ms, the time $T_{pac\_on}$ is set to 94.00 ms (corresponding to the time of 20 base pulses), the pause between the packets is $T_{pac\ off}$ = 55.00 ms, the time $T_{tr\_on}$ is set to 596.00 ms (corresponding to the time of 4 packets) and the pause between the trains is $T_{tr\_off}$ = 600 ms;
    - program 4: saw-tooth base pulse with frequency $f_{imp}$ = 231.48 Hz, where the duration of the base pulse is $T_{imp}$ = 4.32 ms, the time $T_{pac\ on}$ is set to 77.76 ms (corresponding to the time of 18 base pulses), the pause between the packets is $T_{pac\_off}$ = 37.00 ms, the time $T_{tr\_on}$ is set to 459.04 ms (corresponding to the time of 4 packets) and the pause between the trains is $T_{tr\_off}$ = 480 ms;

- program 5: saw-tooth base pulse with frequency $f_{imp}$ = 212.76 Hz, where the duration of the base pulse is $T_{imp}$ = 4.70 ms, the time $T_{pac\,on}$ is set to 75.20 ms (corresponding to the time of 16 base pulses), the pause between the packets is reduced to $T_{pac\_off}$ = 21 ms, the time $T_{tr\_on}$ is set to 384.80 ms (corresponding to the time of 4 packets) and the pause between the trains is $T_{tr\_off}$ = 370 ms;

- program 6: saw-tooth base pulse with frequency $f_{imp}$ = 231.48 Hz, where the duration of the base pulse is $T_{imp}$ = 4.32 ms, the time $T_{pac\,on}$ is set to 60.48 ms (corresponding to the time of 14 base pulses), the pause between the packets is $T_{pac\_off}$ = 16.00 ms, the time $T_{tr\_on}$ is set to 305.92 ms (corresponding to the time of 4 packets) and the pause between the trains is $T_{tr\_off}$ = 350 ms;

- program 7: saw-tooth base pulse with frequency $f_{imp}$ = 212.76 Hz, where the duration of the base pulse is $T_{imp}$ = 4.70 ms, the time $T_{pac\,on}$ is set to 56.40 ms (corresponding to the time of 12 base pulses), the pause between the packets is $T_{pac\_off}$ = 6.60 ms, the time $T_{tr\_on}$ is set to 252.00 ms (corresponding to the time of 4 packets) and the pause between the trains is $T_{tr\_off}$ = 220 ms;

- program 8: saw-tooth base pulse with frequency $f_{imp}$ = 231.48 Hz, where the duration of the base pulse is $T_{imp}$ = 4.32 ms, the time $T_{pac\,on}$ is set to 43.20 ms (corresponding to the time of 10 base pulses), the pause between the packets is $T_{pac\_off}$ = 2.44 ms, the time $T_{tr\_on}$ is set to 183.00 ms (corresponding to the time of 4 packets) and the pause between the trains is $T_{tr\_off}$ = 176 ms; and

- program 9: saw-tooth base pulse with frequency $f_{imp}$ = 189.75 Hz, where the duration of the base pulse is $T_{imp}$ = 5.27 ms, the time $T_{pac\_on}$ is set to 26.35 ms (corresponding to the time of 5 base pulses), the pause between the packets is set to $T_{pac\_off}$ = 16.85 ms, the time $T_{tr\_on}$ is set to 1728.00 ms (corresponding to the time of 40 packets) and the pause between the trains is $T_{tr\_off}$ = 1600 ms.

10. The system for therapeutic treatments according to Claim 8 or Claim 9, wherein said data identifying a treatment program to be executed comprise a program number or the respective timing data.

11. The system for therapeutic treatments according to any of the previous Claims 8 to 10, wherein said further digital processing unit (220a) is configured to:

- receive data identifying a treatment application;
- determine a sequence of a plurality of treatment programs as a function of said data identifying a treatment application;
- starting execution of a first treatment program of said sequence of treatment programs by sending data identifying said first treatment program to said digital processing unit (220b);
- waiting (1006, 1008) that a given treatment program duration has elapsed; and
- selecting the next treatment program of said sequence of treatment programs and starting execution of said next treatment program of said sequence of treatment programs by sending (1002, 1004) data identifying said next treatment program to said digital processing unit (220b).

12. The system for therapeutic treatments according to Claims 11, wherein said further digital processing unit (220a) is configured to receive said data identifying a treatment application via at least one of: a user interface (50), and a communication interface (224a) configured to exchange data with a processing system (10).

13. The system for therapeutic treatments according to any of the previous Claims 8 to 12, wherein said digital processing unit (220b) is an FPGA programmable via respective program data stored to a first non-volatile memory (222b) and/or said further digital processing unit (220a) is a microprocessor programmable via a respective firmware stored to a second non-volatile memory (222a), and wherein said further digital processing unit (220a) is configured to:

- receive new program data for said digital processing unit (220b) and store said new program data to said first non-volatile memory (222b); and/or
- receive a new firmware for said further digital processing unit (220a) and store said new firmware to said second non-volatile memory (222b).

14. The system for therapeutic treatments according to any of the previous claim, wherein said digital processing circuit (220b) comprises a pulse generator (2238) configured to generate a pulsed signal ($PWM_A$) configured to be applied to an acoustic, light and/or vibration transducers, wherein said pulse generator (2238) is configured to set said pulsed signal ($PWM_A$) to high when said second digital value ($S_D$) is greater than a threshold, and to low when said second digital value ($S_D$) is equal to or smaller than said threshold, whereby said pulsed signal ($PWM_A$) has a square waveform synchronized with said supply current ($i_{out}$) according to said given frequency ($f_{imp}$) of said periodic base pulse (I), the switching cycle ($T_{pac}$) of said second PWM signal ($PWM_P$) and the switching cycle ($T_{tr}$) of said third PWM signal ($PWM_{Tr}$).

15. The system for therapeutic treatments according to any of the previous claim, wherein said apparatus (20a) is configured to support a total and/or a local operating mode, wherein:

- in said total operating mode, a first antenna (30) is connected to said apparatus (20a), said first antenna(30) being a planar antenna having a length between 120 cm and 250 cm, preferably between 150 cm and 200 cm, and a width between 40 cm and 120 cm, preferably between 60 cm and 90 cm; and
- in said local operating mode, a second antenna (30) is connected to the apparatus (20a), said second antenna (30) being a planar antenna having a length between 20 cm and 120 cm, preferably, between 20 cm and 60 cm, and a width between 20 cm and 60 cm, or an anatomically modeled antenna.

16. The system for therapeutic treatments according to the combination of Claims 9, 12 and 15, wherein said sequence of a plurality of treatment comprises at least one of:

- *e.g.* for treating multiple sclerosis, a total treatment application consisting in a sequence of treatment programs 1, 3 and 6 and/or a local treatment application consisting in a sequence of treatment programs 3 and 6;
- *e.g.* for treating Parkinson's disease, a total treatment application consisting in a sequence of treatment programs 3, 6 and 3 and/or a first local treatment consisting in a sequence of treatment programs 3 and 6 and/or a second local treatment application consisting in a sequence of treatment programs 8 and 8;
- *e.g.* for treating Alzheimer's disease or senile dementia, a total treatment application consisting in a sequence of treatment programs 3, 6 and 9 and/or a local treatment application consisting in a sequence of treatment programs 3 and 6;
- *e.g.* for treating postictal states, a total treatment application consisting in a sequence of treatment programs 3, 3 and 6 and/or a local treatment application consisting in a sequence of treatment programs 6 and 6;
- *e.g.* for treating fibromyalgia or chronic fatigue syndrome, a total treatment application consisting in a sequence of treatment programs 3, 6 and 9 and/or a local treatment application consisting in a sequence of treatment programs 6 and 8; and
- *e.g.* for treating the Psycho-Neuro-Endocrine-Immunology (P.N.E.I.) system, a total treatment application consisting in a sequence of treatment programs 9, 6 and 9 and/or a local treatment application consisting in a sequence of treatment programs 3 and 6.

**Patentansprüche**

1. System für therapeutische Behandlungen mit elektromagnetischen Wellen, umfassend eine Antenne (30) und eine Vorrichtung (20a), die so konfiguriert ist, dass sie einen Versorgungsstrom ($i_{out}$) für die Antenne (30) erzeugt, um die elektromagnetische Welle zu erzeugen, wobei die Vorrichtung (20a) umfasst:

- eine digitale Verarbeitungsschaltung (220b), die so konfiguriert ist, dass sie ein erstes PWM-Signal (DRV) mit einem gegebenen Arbeitszyklus erzeugt, wobei das erste PWM-Signal (DRV) für jeden Schaltzyklus ($T_{sw}$) für eine Einschaltperiode ($T_{ON}$) auf High und für eine Ausschaltperiode (Torr) auf Low gesetzt wird;
- eine Schaltstufe (24a), die so konfiguriert ist, dass sie ein verstärktes PWM-Signal ($V_{240}$) durch Verstärken des ersten PWM-Signals (DRV) erzeugt;
- einen analogen Tiefpass- oder Bandpassfilter (28), der so konfiguriert ist, dass es den Versorgungsstrom ($i_{out}$) durch Filtern des verstärkten PWM-Signals ($V_{240}$) erzeugt;
- einen Stromsensor (228), der so konfiguriert ist, dass er einen digitalen Abtastwert ($CS_D$) liefert, der die Amplitude des Versorgungsstroms ($i_{out}$) anzeigt;

wobei die digitale Verarbeitungsschaltung (220b) konfiguriert ist, um:

- eine Folge von ersten digitalen Werten ($S_{imp}$) eines periodischen Basisimpulses (I) mit einer gegebenen Frequenz ($f_{imp}$) zu erzeugen (2224);
- Erzeugen (2228) eines zweiten PWM-Signals ($PWM_P$), wobei das zweite PWM-Signal ($PWM_P$) für jeden Schaltzyklus ($T_{pac}$) auf ein entsprechendes erstes Logiklevel für eine Paketeinschaltperiode ($T_{pac\_on}$) und auf ein entsprechendes zweites Logiklevel für eine Paketausschaltperiode ($T_{pac\_off}$) gesetzt wird;

- Erzeugen (2230) eines dritten PWM-Signals (PWM$_{Tr}$), wobei das dritte PWM-Signal (PWM$_{Tr}$) für jeden Schaltzyklus (T$_{tr}$) auf einen jeweiliges erstes Logiklevel für eine FolgeEinschaltperiode (T$_{tr\_on}$) und auf ein jeweiliges zweites Logiklevel für eine FolgeAusschaltperiode (T$_{tr\_off}$) gesetzt wird;
- Erzeugen (2232) eines Freigabesignals (EN), wobei das Freigabesignal (EN) auf ein entsprechendes erstes Logiklevel gesetzt wird, wenn das zweite PWM-Signal (PWM$_P$) das entsprechende erste Logiklevel hat und das dritte PWM-Signal (PWM$_{Tr}$) das jeweilige erste Logiklevel hat, und auf ein jeweiliges zweites Logiklevel gesetzt wird, wenn das zweite PWM-Signal (PWM$_P$) das jeweilige zweite Logiklevel hat oder das dritte PWM-Signal (PWM$_{Tr}$) das jeweilige zweite Logiklevel hat;
- Erzeugen (2234) eines zweiten digitalen Wertes (S$_D$), wobei der zweite digitale Wert (S$_D$) auf den ersten digitalen Wert (S$_{imp}$) gesetzt wird, wenn das Freigabesignal (EN) das jeweilige erste Logiklevel hat und auf Null gesetzt wird, wenn das Freigabesignal (EN) das jeweilige zweite Logiklevel hat;
- Erzeugen (2238) des ersten PWM-Signals (DRV) als eine Funktion von einem dritten digitalen Wert (D), der den gegebenen Arbeitszyklus anzeigt;
- Variieren des dritten digitalen Wertes (D) über eine diskrete Proportional-Integral-Regelung, die so konfiguriert ist, dass sie die Differenz zwischen dem zweiten digitalen Wert (S$_D$) und dem digitalen Abtastwert (CS$_D$) auf Null reguliert.

2. System für therapeutische Behandlungen nach Anspruch 1, wobei die digitale Verarbeitungsschaltung (220b) mit einem FPGA oder einem ASIC implementiert ist, umfassend:

   - einen digitalen Signalgenerator (2224), der so konfiguriert ist, dass er die Folge der ersten digitalen Werte (S$_{imp}$) erzeugt;
   - eine erste digitale PWM-Generatorschaltung (2238), die so konfiguriert ist, dass sie das erste PWM-Signal (PWM$_P$) als Funktion eines dritten digitalen Werts (D) erzeugt, der den gegebenen Arbeitszyklus angibt;
   - eine zweite digitale PWM-Generatorschaltung (2228), die zum Erzeugen des zweiten PWM-Signals (PWM$_P$) konfiguriert ist;
   - eine dritte digitale PWM-Generatorschaltung (2230), die zum Erzeugen des dritten PWM-Signals (PWM$_{Tr}$) konfiguriert ist;
   - ein erstes Logikgatter (2232), das so konfiguriert ist, dass es das Freigabesignal (EN) als eine Funktion von dem zweiten PWM-Signal (PWM$_P$) und dem dritten PWM-Signal (PWM$_{Tr}$) erzeugt;
   - eine zweite Logikschaltung (2234), die so konfiguriert ist, dass sie den zweiten digitalen Wert (S$_D$) als eine Funktion des ersten digitalen Wertes (S$_{imp}$) und des Freigabesignals (EN) erzeugt;
   - eine diskrete Proportional-Integral-Reglerschaltung (2236), die so konfiguriert ist, dass sie den dritten digitalen Wert (D) als eine Funktion des zweiten digitalen Wert (S$_D$) und des digitalen Abtastwert (CS$_D$) verändert.

3. System für therapeutische Behandlungen nach Anspruch 2, wobei der digitale Signalgenerator (2224) so konfiguriert ist, dass er periodische Basisimpulse (I) mit einem Sägezahnprofil erzeugt, und wobei der digitale Signalgenerator (2224) einen Zähler (2260) umfasst, der konfiguriert ist:

   - in Reaktion auf ein Taktsignal (CLK) mit einer gegebenen Frequenz (f$_{CLK}$) die ersten digitalen Werte (S$_{imp}$) um einen Inkrementwert (INC) zu erhöhen; und
   - die ersten digitalen Werte (S$_{imp}$) zurückzusetzen, wenn die ersten digitalen Werte (S$_{imp}$) einen vorgegebenen Maximalwert (S$_{Dmax}$) erreichen.

4. System für therapeutische Behandlungen nach Anspruch 3, wobei der digitale Signalgenerator (2224) eine Inkrementsteuerschaltung (2262) umfasst, die so konfiguriert ist, dass sie den Inkrementwert (INC) als eine Funktion von:

   - Daten zur Identifizierung der Frequenz (f$_{CLK}$) oder des Taktsignals (CLK);
   - Daten zur Identifizierung der Frequenz (f$_{imp}$) der periodischen Basisimpulse (I);
   - dem Höchstwert (S$_{DmaX}$).

5. System für therapeutische Behandlungen nach Anspruch 2, wobei der digitale Signalgenerator (2224) umfasst:

   - eine Nachschlagetabelle (2254) mit einer Vielzahl von Elementen, die mit einer gegebenen Anzahl von Abtastwerten einer Standardwellenform der Basisimpulse (I) mit einer gegebenen Standardfrequenz (fs) korrespondieren, wobei jedes Element die Amplitude {A$_k$) einer jeweiligen Abtastung der Standardwellenform der Basisimpulse (I) gespeichert hat, wobei die Nachschlagetabelle (2254) am Eingang einen Zählwert empfängt, der einen gegebenen Abtastwert auswählt, und wobei der erste digitale Wert (S$_{imp}$) als eine Funktion der

Amplitude ($A_k$) des jeweiligen ausgewählten Abtastwerts bestimmt wird;
- einen Zähler (2252), der so konfiguriert ist, dass er in Reaktion auf ein Taktsignal (CLK) den Zählwert um einen Inkrementwert (INC) erhöht, wobei der Zähler (2252) einen Phasenakkumulator darstellt; und
- eine Inkrementsteuerschaltung (2258), die so konfiguriert ist, dass sie den Inkrementwert (INC) als eine Funktion von Daten, die die Standardfrequenz (fs) identifizieren, und von Daten, die die Frequenz ($f_{imp}$) der periodischen Basisimpulse (I) identifizieren, erzeugt.

6. System für therapeutische Behandlungen nach Anspruch 4 oder Anspruch 5, wobei die Inkrementsteuerschaltung (2262; 2258) so konfiguriert ist, dass sie einen internen Inkrementwert (INC') berechnet, wobei der interne Inkrementwert (INC') einen ganzzahligen Teil mit einer Anzahl von Bits, die der Anzahl von Bits des Inkrementwerts (INC) entspricht, und einen fraktalen Teil aufweist, und wobei die Inkrementsteuerschaltung (2262; 2258) so konfiguriert ist, dass sie den Inkrementwert (INC) so verändert, dass der Inkrementwert (INC) einen Durchschnittswert aufweist, der mit dem internen Inkrementwert (INC') korrespondiert.

7. System für therapeutische Behandlungen nach einem der vorhergehenden Ansprüche, wobei die digitale Verarbeitungsschaltung (220b) eine dritte digitale PWM-Generatorschaltung (2242) umfasst, die so konfiguriert ist, dass sie ein Polaritätssignal (POL) erzeugt, und wobei die Schaltstufe (24a) umfasst:

- eine erste Halbbrücke mit einem ersten (SW1) und einem zweiten (SW2) elektronischen Schalter, wobei der Zwischenknoten zwischen dem ersten (SW1) und dem zweiten (SW2) elektronischen Schalter ein erster Schaltknoten ist;
- eine zweite Halbbrücke mit einem dritten (SW3) und einem vierten (SW4) elektronischen Schalter, wobei der Zwischenknoten zwischen dem dritten (SW3) und dem vierten (SW4) elektronischen Schalter ein zweiter Schaltknoten ist, wobei das verstärkte PWM-Signal ($V_{240}$) mit der Spannung zwischen dem ersten Schaltknoten und dem zweiten Schaltknoten korrespondiert;
- eine Treiberschaltung (242), die so konfiguriert ist, dass sie Treibersignale (DRV1-DRV4) für den ersten (SW1), zweiten (SW2), dritten (SW3) und vierten (SW4) elektronischen Schalter als eine Funktion des ersten PWM-Signals (DRV) und des Polaritätssignals (POL) erzeugt.

8. System für therapeutische Behandlungen nach einem der vorhergehenden Ansprüche, wobei die digitale Verarbeitungsschaltung (220b) umfasst:

- eine erste Kommunikationsschnittstelle (2220) zum Austausch von Daten mit einer weiteren digitalen Verarbeitungseinheit (220a); und
- eine Steuerschaltung (2222), die so konfiguriert ist, dass sie über die erste Kommunikationsschnittstelle (2220) Daten empfängt, die ein auszuführendes Behandlungsprogramm identifizieren, und als eine Funktion von diesen Daten, die ein auszuführendes Behandlungsprogramm identifizieren, bestimmen:

  - die Frequenz ($f_{imp}$) des periodischen Basisimpulses (I),
  - die Paketeinschaltperiode ($T_{pac\_on}$) und die Paketausschaltperiode ($T_{pac\_off}$), und
  - die Folgeeinschaltperiode ($T_{tr\_on}$) und die Folgeausschaltperiode ($T_{tr\_off}$).

9. System für therapeutische Behandlungen nach Anspruch 8, wobei die digitale Verarbeitungsschaltung (220b) und die weitere digitale Verarbeitungseinheit (220a) so konfiguriert sind, dass sie mindestens eines der folgenden Behandlungsprogramme ausführen:

- Programm 1: Sägezahn-Basisimpuls mit der Frequenz $f_{imp}$ = 212,76 Hz, wobei die Dauer des des Basisimpulses $T_{imp}$ = 4,70 ms beträgt, die Zeit $T_{pac\ on}$ auf 206,80 ms eingestellt ist, (entsprechend der Zeit von 44 Basisimpulsen), die Pause zwischen den Paketen $T_{pac\_off}$ = 140,00 ms ist, die Zeit $T_{tr\_on}$ auf 1387,20 ms eingestellt wird (entsprechend der Zeit von 4 Paketen) und die Pause zwischen den Folgen $T_{tr\_off}$ = 1450 ms beträgt;
- Programm 2: Sägezahn-Basisimpuls mit der Frequenz $f_{imp}$ = 231,48 Hz, wobei die Dauer des des Basisimpulses $T_{imp}$ = 4,32 ms beträgt, die Zeit $T_{pac\_on}$ auf 146,88 ms gesetzt wird (entsprechend der Zeit der 34 Basisimpulse), die Pause zwischen den Paketen $T_{pac\_off}$ = 105,00 ms ist, die Zeit $T_{tr\_on}$ auf 1007,52 ms (entsprechend der Zeit von 4 Paketen) und die Pause zwischen den Folgen $T_{tr\_off}$ = 1100 ms beträgt;
- Programm 3: Sägezahn-Basisimpuls mit der Frequenz $f_{imp}$ = 212,76 Hz, wobei die Dauer des Basisimpulses $T_{imp}$ = 4,70 ms ist, die Zeit $T_{pac\_on}$ auf 94,00 ms (entsprechend der Zeit von 20 Basisimpulsen) eingestellt wird, die Pause zwischen den Paketen $T_{pac\_off}$ = 55,00 ms, die Zeit $T_{tr\_on}$ auf 596,00 ms eingestellt wird (entsprechend der Zeit von 4 Paketen) und die Pause zwischen den Folgen $T_{tr\_off}$ = 600 ms ist;

- Programm 4: Sägezahn-Basisimpuls mit der Frequenz $f_{imp}$ = 231,48 Hz, wobei die Dauer des Basisimpulses $T_{imp}$ = 4,32 ms, die Zeit $T_{pac\_on}$ auf 77,76 ms (entsprechend der Zeit von 18 Basisimpulsen) eingestellt wird, die Pause zwischen den Paketen $T_{pac\_off}$ = 37,00 ms ist, die Zeit auf $T_{tr\_on}$ = 459,04 eingestellt wird (entsprechend der Zeit von 4 Paketen) und die Pause zwischen den Folgen $T_{tr\_off}$ = 480 ms beträgt;

- Programm 5: Sägezahn-Basisimpuls mit der Frequenz $f_{imp}$ = 212,76 Hz, wobei die Dauer des Basisimpulses $T_{imp}$ = 4,70 ms beträgt, die Zeit $T_{pac\_on}$ auf 75,20 ms eingestellt ist (entsprechend der Zeit von 16 Basisimpulsen), die Pause zwischen den Paketen auf $T_{pac\_off}$ = 21 ms reduziert wird, die Zeit $T_{tr\_on}$ auf 384,80 ms eingestellt wird (entsprechend der Zeit von 4 Paketen) und die Pause zwischen den Folgen $T_{tr\_off}$ = 370 ms beträgt;

- Programm 6: Sägezahn-Basisimpuls mit der Frequenz $f_{imp}$ = 231,48 Hz, wobei die Dauer des Basisimpulses $T_{imp}$ = 4,32 ms ist, die Zeit $T_{pac\_on}$ auf 60,48 ms gesetzt wird (entsprechend der Zeit von 14 Basisimpulsen), die Pause zwischen den Paketen $T_{pac\_off}$ = 16.00 ms ist, die Zeit $T_{tr\_on}$ auf 305.92 ms eingestellt wird (entsprechend der Zeit von 4 Paketen) und die Pause zwischen den Folgen $T_{tr\_off}$ = 350 ms beträgt;

- Programm 7: Sägezahn-Basisimpuls mit der Frequenz $f_{imp}$ = 212,76 Hz, wobei die Dauer des Basisimpulses $T_{imp}$ = 4,70 ms ist, die Zeit $T_{pac\_on}$ auf 56,40 ms eingestellt wird (entsprechend der Zeit von 12 Basisimpulsen), die Pause zwischen den Paketen $T_{pac\_off}$ = 6,60 ms, die Zeit $T_{tr\_on}$ auf 252,00 ms eingestellt wird (entsprechend der Zeit von 4 Paketen) und die Pause zwischen den Folgen $T_{tr\_off}$ = 220 ms beträgt;

- Programm 8: Sägezahn-Basisimpuls mit der Frequenz $f_{imp}$ = 231,48 Hz, wobei die Dauer des Basisimpulses $T_{imp}$ = 4,32 ms ist, die Zeit $T_{pac\_on}$ auf 43,20 ms gesetzt ist (entsprechend der Zeit der 10 Basisimpulse), die Pause zwischen den Paketen ist $T_{pac\_off}$ = 2,44 ms, die Zeit $T_{tr\_on}$ wird auf 183,00 ms eingestellt (entsprechend der Zeit von 4 Paketen) und die Pause zwischen den Folgen auf $T_{tr\_off}$ = 176 ms eingestellt wird; und

- Programm 9: Sägezahn-Basisimpuls mit der Frequenz $f_{imp}$ = 189,75 Hz, wobei die Dauer des Basisimpulses $T_{imp}$ = 5,27 ms beträgt, die Zeit $T_{pac\_on}$ auf 26,35 ms eingestellt ist (entsprechend der Zeit von 5 Basisimpulse), die Pause zwischen den Paketen auf $T_{pac\_off}$ = 16,85 ms eingestellt wird, die Zeit $T_{tr\_on}$ auf 1728,00 ms eingestellt wird (entsprechend der Zeit von 40 Paketen) und die Pause zwischen den Folgen $T_{tr\_off}$ = 1600 ms ist.

10. System für therapeutische Behandlungen nach Anspruch 8 oder Anspruch 9, wobei die Daten, die ein auszuführendes Behandlungsprogramm identifizieren, eine Programmnummer oder die entsprechenden Zeitdaten umfassen.

11. System für therapeutische Behandlungen nach einem der vorhergehenden Ansprüche 8 bis 10, wobei die weitere digitale Verarbeitungseinheit (220a) konfiguriert ist, um:

- Daten zu empfangen, die eine Behandlungsanwendung identifizieren;
- eine Sequenz einer Vielzahl von Behandlungsprogrammen als eine Funktion von den eine Behandlungsanwendung identifizierenden Daten zu bestimmen;
- Starten der Ausführung eines ersten Behandlungsprogramms der Sequenz von Behandlungsprogrammen durch Senden von Daten, die das erste Behandlungsprogramm identifizieren, an die digitale Verarbeitungseinheit (220b);
- Warten (1006, 1008), dass eine gegebene Behandlungsprogrammdauer abgelaufen ist; und
- Auswählen des nächsten Behandlungsprogramms der Folge von Behandlungsprogrammen und Starten der Ausführung des nächsten Behandlungsprogramms der Folge von Behandlungsprogrammen durch Senden (1002, 1004) von Daten, die das nächste Behandlungsprogramm identifizieren, an die digitale Verarbeitungseinheit (220b).

12. System für therapeutische Behandlungen nach Anspruch 11, wobei die weitere digitale Verarbeitungseinheit (220a) so konfiguriert ist, dass sie die Daten, die eine Behandlungsanwendung identifizieren, über mindestens eines von Folgendem empfängt: einer Benutzerschnittstelle (50) und eine Kommunikationsschnittstelle (224a), die so konfiguriert ist, dass sie Daten mit einem Verarbeitungssystem (10) austauscht.

13. System für therapeutische Behandlungen nach einem der vorhergehenden Ansprüche 8 bis 12, wobei die digitale Verarbeitungseinheit (220b) ein FPGA ist, das über entsprechende Programmdaten programmierbar ist, die in einem ersten nichtflüchtigen Speicher (222b) gespeichert sind, und/oder die weitere digitale Verarbeitungseinheit (220a) ein Mikroprozessor ist, der über eine entsprechende Firmware programmierbar ist, die in einem zweiten nichtflüchtigen Speicher (222a) gespeichert ist, und wobei die weitere digitale Verarbeitungseinheit (220a) konfiguriert ist, um:

- neue Programmdaten für die digitale Verarbeitungseinheit (220b) zu empfangen und die neuen Programmdaten in dem ersten nicht-flüchtigen Speicher (222b) zu speichern; und/oder

- eine neue Firmware für die weitere digitale Verarbeitungseinheit (220a) zu empfangen und die neue Firmware in dem zweiten nicht-flüchtigen Speicher (222b) zu speichern.

14. System für therapeutische Behandlungen nach einem der vorhergehenden Ansprüche, wobei die digitale Verarbeitungsschaltung (220b) einen Impulsgenerator (2238) umfasst, der so konfiguriert ist, dass er ein gepulstes Signal (PWM$_A$) erzeugt, das so konfiguriert ist, dass es auf einen Akustik-, Licht- und/oder Vibrationswandler angewendet wird, wobei der Impulsgenerator (2238) so konfiguriert ist, dass er das gepulste Signal (PWM$_A$) auf High setzt, wenn der zweite digitale Wert (S$_D$) größer als ein Schwellenwert ist, und auf Low setzt, wenn der zweite digitale Wert (S$_D$) gleich oder kleiner als der Schwellenwert ist, wobei das gepulste Signal (PWM$_A$) eine rechteckige Wellenform hat, die mit dem Versorgungsstrom (i$_{out}$) gemäß der gegebenen Frequenz (f$_{imp}$) des periodischen Basisimpulses (I), dem Schaltzyklus (T$_{pac}$) des zweiten PWM-Signals (PWM$_P$) und dem Schaltzyklus (T$_{tr}$) des dritten PWM-Signals (PWM$_{Tr}$) synchronisiert ist.

15. System für therapeutische Behandlungen nach einem der vorhergehenden Ansprüche, wobei die Vorrichtung (20a) so konfiguriert ist, dass sie einen Gesamt- und/oder einen lokalen Betriebsmodus unterstützt, wobei:

- in dem Gesamtbetriebsmodus eine erste Antenne (30) mit der Vorrichtung (20a) verbunden ist, wobei die erste Antenne (30) eine Planar-Antenne ist, die eine Länge zwischen 120 cm und 250 cm, vorzugsweise zwischen 150 cm und 200 cm, und einer Breite zwischen 40 cm und 120 cm, vorzugsweise zwischen 60 cm und 90 cm hat; und
- in dem lokalen Betriebsmodus eine zweite Antenne (30) mit der Vorrichtung (20a) verbunden ist, wobei die zweite Antenne (30) eine Planar-Antenne mit einer Länge zwischen 20 cm und 120 cm ist, vorzugsweise zwischen 20 cm und 60 cm, und einer Breite zwischen 20 cm und 60 cm, oder eine anatomisch modellierte Antenne.

16. System für therapeutische Behandlungen gemäß der Kombination der Ansprüche 9, 12 und 15, wobei die Sequenz einer Vielzahl von Behandlungen mindestens eine der folgenden umfasst:

- z.B. zur Behandlung von Multipler Sklerose, eine Gesamtbehandlungsanwendung, bestehend aus einer Abfolge von Behandlungsprogrammen 1, 3 und 6 und/oder eine lokale Behandlungsanwendung, bestehend aus einer Abfolge von Behandlungsprogrammen 3 und 6;
- z.B. zur Behandlung der Parkinson-Krankheit, eine Gesamtbehandlungsanwendung, die aus einer Abfolge der Behandlungsprogramme 3, 6 und 3 besteht und/oder eine erste lokale Behandlung, die aus einer Abfolge der Behandlungsprogramme 3 und 6 besteht und/oder eine zweite lokale Behandlungsanwendung, die aus einer Abfolge der Behandlungsprogramme 8 und 8 besteht;
- z.B. zur Behandlung der Alzheimer-Krankheit oder der senilen Demenz, eine Gesamtbehandlungsanwendung, die aus einer Abfolge der Behandlungsprogramme 3, 6 und 9 besteht und/oder eine lokale Behandlungsanwendung, die aus einer Abfolge der Behandlungsprogramme 3 und 6 besteht;
- z.B. zur Behandlung postiktaler Zustände, eine Gesamtbehandlungsanwendung, die aus einer Abfolge der Behandlungsprogramme 3, 3 und 6 besteht, und/oder eine lokale Behandlungsanwendung, die aus einer Abfolge der Behandlungsprogramme 6 und 6 besteht;
- z.B. zur Behandlung der Fibromyalgie oder des chronischen Müdigkeitssyndroms, eine Gesamtbehandlungsanwendung, die aus einer Abfolge der Behandlungsprogramme 3, 6 und 9 besteht, und/oder eine lokale Behandlungsanwendung, die aus einer Abfolge der Behandlungsprogramme 6 und 8 besteht, und
- z.B. zur Behandlung des Psycho-Neuro-Endokrin-Immunologie (P.N.E.I.) Systems, eine Gesamtbehandlungsanwendung, die aus einer Abfolge der Behandlungsprogramme 9, 6 und 9 besteht, und/oder eine lokale Behandlungsanwendung, die aus einer Abfolge der Behandlungsprogramme 3 und 6 besteht.

**Revendications**

1. Système de traitement thérapeutique par ondes électromagnétiques, comprenant une antenne (30) et un appareil (20a) configuré pour générer un courant d'alimentation (i$_{out}$) pour ladite antenne (30) afin de générer ladite onde électromagnétique, dans lequel ledit appareil (20a) comprend :

- un circuit de traitement numérique (220b) configuré pour générer un premier signal PWM (DRV) ayant un rapport cyclique donné, dans lequel, pour chaque cycle de commutation (Tsw), ledit premier signal PWM (DRV) est réglé sur haut pendant une période d'activation (T$_{ON}$) et sur bas pendant une période de désactivation (T$_{OFF}$) ;

- un étage de commutation (24a) configuré pour générer un signal PWM amplifié ($V_{240}$) en amplifiant ledit premier signal PWM (DRV) ;
- un filtre analogique passe-bas ou passe-bande (28) configuré pour générer ledit courant d'alimentation ($i_{out}$) en filtrant ledit signal PWM amplifié ($V_{240}$) ;
- un capteur de courant (228) configuré pour fournir un échantillon numérique ($CS_D$) indiquant l'amplitude dudit courant d'alimentation ($i_{out}$) ;

dans lequel ledit circuit de traitement numérique (220b) est configuré pour :

- générer (2224) une séquence de premières valeurs numériques ($S_{imp}$) d'une impulsion de base (I) périodique ayant une fréquence ($f_{imp}$) donnée ;
- générer (2228) un deuxième signal PWM ($PWM_P$), dans lequel, pour chaque cycle de commutation ($T_{pac}$), ledit deuxième signal PWM ($PWM_P$) est réglé sur un premier niveau logique respectif pendant une période d'activation de paquet ($T_{pac\_on}$) et sur un deuxième niveau logique respectif pendant une période de désactivation de paquet ($T_{pac\_off}$) ;
- générer (2230) un troisième signal PWM ($PWM_{Tr}$), dans lequel, pour chaque cycle de commutation ($T_{tr}$), ledit troisième signal PWM ($PWM_{Tr}$) est réglé sur un premier niveau logique respectif pendant une période d'activation de train ($T_{tr\_on}$) et sur un deuxième niveau logique respectif pendant une période de désactivation de train ($T_{tr\_off}$) ;
- générer (2232) un signal de validation (EN), dans lequel ledit signal de validation (EN) est réglé sur un premier niveau logique respectif lorsque ledit deuxième signal PWM ($PWM_P$) est au premier niveau logique respectif et que ledit troisième signal PWM ($PWM_{Tr}$) est au premier niveau logique respectif, et sur un deuxième niveau logique respectif lorsque ledit deuxième signal PWM ($PWM_P$) est au deuxième niveau logique respectif ou que ledit troisième signal PWM ($PWM_{Tr}$) est au deuxième niveau logique respectif ;
- générer (2234) une deuxième valeur numérique ($S_D$), dans lequel la deuxième valeur numérique ($S_D$) est réglée sur la première valeur numérique ($S_{imp}$) lorsque ledit signal de validation (EN) est au premier niveau logique respectif, et sur zéro lorsque ledit signal de validation (EN) est au deuxième niveau logique respectif ;
- générer (2238) ledit premier signal PWM (DRV) en fonction d'une troisième valeur numérique (D) indiquant ledit rapport cyclique donné ;
- faire varier ladite troisième valeur numérique (D) via une régulation proportionnelle-intégrale discrète configurée pour réguler la différence entre ladite deuxième valeur numérique ($S_D$) et ledit échantillon numérique ($CS_D$) à zéro.

**2.** Système de traitement thérapeutique selon la revendication 1, dans lequel ledit circuit de traitement numérique (220b) est mis en oeuvre avec un FPGA ou un ASIC comprenant :

- un générateur de signaux numériques (2224) configuré pour générer ladite séquence de premières valeurs numériques ($S_{imp}$) ;
- un premier circuit générateur PWM numérique (2238) configuré pour générer ledit premier signal PWM ($PWM_P$) en fonction de ladite troisième valeur numérique (D) indiquant ledit rapport cyclique donné ;
- un deuxième circuit générateur PWM numérique (2228) configuré pour générer ledit deuxième signal PWM ($PWM_P$) ;
- un troisième circuit générateur PWM numérique (2230) configuré pour générer ledit troisième signal PWM ($PWM_{Tr}$) ;
- une première porte logique (2232) configurée pour générer ledit signal de validation (EN) en fonction dudit deuxième signal PWM ($PWM_P$) et dudit troisième signal PWM ($PWM_{Tr}$) ;
- un deuxième circuit logique (2234) configuré pour générer ledit deuxième valeur numérique ($S_D$) en fonction de ladite première valeur numérique ($S_{imp}$) et dudit signal de validation (EN) ;
- un circuit régulateur proportionnel-intégral discret (2236) configuré pour faire varier ladite troisième valeur numérique (D) en fonction de ladite deuxième valeur numérique ($S_D$) et dudit échantillon numérique ($CS_D$)

**3.** Système de traitement thérapeutique selon la revendication 2, dans lequel ledit générateur de signaux numériques (2224) est configuré pour générer des impulsions de base (I) périodiques ayant un profil en dents de scie, et dans lequel ledit générateur de signaux numériques (2224) comprend un compteur (2260) configuré pour :

- en réponse au fait qu'un signal d'horloge (CLK) a une fréquence ($f_{CLK}$) donnée, augmenter lesdites premières valeurs numériques ($S_{imp}$) d'une valeur d'incrémentation (INC) ; et
- réinitialiser lesdites premières valeurs numériques ($S_{imp}$) lorsque lesdites premières valeurs numériques ($S_{imp}$)

atteignent une valeur maximale ($S_{Dmax}$) donnée.

4. Système de traitement thérapeutique selon la revendication 3, dans lequel ledit générateur de signaux numériques (2224) comprend un circuit de commande d'incrémentation (2262) configuré pour générer ladite valeur d'incrémentation (INC) en fonction de :

   - données identifiant ladite fréquence ($f_{CLK}$) ou ledit signal d'horloge (CLK) ;
   - données identifiant ladite fréquence ($f_{imp}$) desdites impulsions de base (I) périodiques ;
   - ladite valeur maximale ($S_{Dmax}$).

5. Système de traitement thérapeutique selon la revendication 2, dans lequel ledit générateur de signaux numériques (2224) comprend :

   - une table de consultation (2254) comprenant une pluralité d'éléments correspondant à un nombre donné d'échantillons d'une forme d'onde standard desdites impulsions de base (I) avec une fréquence standard (fs) donnée, dans lequel chaque élément a stocké l'amplitude ($A_k$) d'un échantillon respectif de ladite forme d'onde standard desdites impulsions de base (I), dans lequel ladite table de consultation (2254) reçoit en entrée une valeur de comptage sélectionnant un échantillon donné, et dans lequel ladite première valeur numérique ($S_{imp}$) est déterminée en fonction de l'amplitude ($A_k$) de l'échantillon sélectionné respectif ;
   - un compteur (2252) configuré pour, en réponse à un signal d'horloge (CLK), augmenter ladite valeur de comptage d'une valeur d'incrémentation (INC), ledit compteur (2252) représentant un accumulateur de phase ; et
   - un circuit de commande d'incrémentation (2258) configuré pour générer ladite valeur d'incrémentation (INC) en fonction de données identifiant ladite fréquence standard (fs) et de données identifiant ladite fréquence ($f_{imp}$) desdites impulsions de base (I) périodiques.

6. Système de traitement thérapeutique selon la revendication 4 ou la revendication 5, dans lequel ledit circuit de commande d'incrémentation (2262 ; 2258) est configuré pour calculer une valeur d'incrémentation interne (INC'), ladite valeur d'incrémentation interne (INC') ayant une partie entière ayant un nombre de bits correspondant au nombre de bits de ladite valeur d'incrémentation (INC), et une partie fractionnaire, et dans lequel ledit circuit de commande d'incrémentation (2262 ; 2258) est configuré pour faire varier ladite valeur d'incrémentation (INC), de sorte que ladite valeur d'incrémentation (INC) ait une valeur moyenne correspondant à ladite valeur d'incrémentation interne (INC').

7. Système de traitement thérapeutique selon l'une des revendications précédentes, dans lequel ledit circuit de traitement numérique (220b) comprend un troisième circuit générateur PWM numérique (2242) configuré pour générer un signal de polarité (POL), et dans lequel ledit étage de commutation (24a) comprend :

   - une première demi-bride comprenant un premier (SW1) et un deuxième (SW2) commutateur électronique, dans lequel le noeud intermédiaire entre ledit premier (SW1) et ledit deuxième (SW2) commutateur électronique est un premier noeud de commutation ;
   - une deuxième demi-bride comprenant un troisième (SW3) et un quatrième (SW4) commutateur électronique, dans lequel le noeud intermédiaire entre ledit troisième (SW3) et ledit quatrième (SW4) commutateur électronique est un deuxième noeud de commutation, dans lequel ledit signal PWM amplifié ($V_{240}$) correspond à la tension entre ledit premier noeud de commutation et ledit deuxième noeud de commutation ;
   - un circuit d'excitation (242) configuré pour générer signaux d'excitation (DRV1-DRV4) pour lesdits premier (SW1), deuxième (SW2), troisième (SW3) et quatrième (SW4) commutateurs électroniques en fonction dudit premier signal PWM (DRV) et dudit signal de polarité (POL).

8. Système de traitement thérapeutique selon l'une des revendications précédentes, dans lequel ledit circuit de traitement numérique (220b) comprend :

   - une première interface de communication (2220) pour l'échange de données avec une unité de traitement numérique (220a) supplémentaire ; et
   - un circuit de commande (2222) configuré pour recevoir, via ladite première interface de communication (2220), des données identifiant un programme de traitement à exécuter, et en fonction desdites données identifiant un programme de traitement à exécuter, déterminer :
   - ladite fréquence ($f_{imp}$) de ladite impulsion de base (I) périodique,
   - ladite période d'activation de paquet ($T_{pac\_on}$) et ladite période de désactivation de paquet ($T_{pac\_off}$), et

- ladite période d'activation de train ($T_{tr\_on}$) et ladite période de désactivation de train ($T_{tr\_off}$).

9. Système de traitement thérapeutique selon la revendication 8, dans lequel ledit circuit de traitement numérique (220b) et ladite unité de traitement numérique (220a) supplémentaire sont configurés pour exécuter au moins l'un des programmes de traitement suivants :

- programme 1 : impulsion de base en dents de scie avec une fréquence $f_{imp}$ = 212,76 Hz, où la durée de l'impulsion de base est $T_{imp}$ = 4,70 ms, le temps $T_{pac\_on}$ est réglé sur 206,80 ms (correspondant au temps de 44 impulsions de base), la pause entre les paquets est $T_{pac\_off}$ = 140,00 ms, le temps $T_{tr\_on}$ est réglé sur 1387,20 ms (correspondant au temps de 4 paquets) et la pause entre les trains est $T_{tr\_off}$ = 1450 ms ;
- programme 2 : impulsion de base en dents de scie avec une fréquence $f_{imp}$ = 231,48 Hz, où la durée de l'impulsion de base est $T_{imp}$ = 4,32 ms, le temps $T_{pac\_on}$ est réglé sur 146,88 ms (correspondant au temps de 34 impulsions de base), la pause entre les paquets est $T_{pac\_off}$ = 105,00 ms, le temps $T_{tr\_on}$ est réglé sur 1007,52 ms (correspondant au temps de 4 paquets) et la pause entre les trains est $T_{tr\_off}$ = 1100 ms ;
- programme 3 : impulsion de base en dents de scie avec une fréquence $f_{imp}$ = 212,76 Hz, où la durée de l'impulsion de base est $T_{imp}$ = 4,70 ms, le temps $T_{pac\_on}$ est réglé sur 94,00 ms (correspondant au temps de 20 impulsions de base), la pause entre les paquets est $T_{pac\_off}$ = 55,00 ms, le temps $T_{tr\_on}$ est réglé sur 596,00 ms (correspondant au temps de 4 paquets) et la pause entre les trains est $T_{tr\_off}$ = 600 ms ;
- programme 4 : impulsion de base en dents de scie avec une fréquence $f_{imp}$ = 231,48 Hz, où la durée de l'impulsion de base est $T_{imp}$ = 4,32 ms, le temps $T_{pac\_on}$ est réglé sur 77,76 ms (correspondant au temps de 18 impulsions de base), la pause entre les paquets est $T_{pac\_off}$ = 37,00 ms, le temps $T_{tr\_on}$ est réglé sur 459,04 ms (correspondant au temps de 4 paquets) et la pause entre les trains est $T_{tr\_off}$ = 480 ms ;
- programme 5 : impulsion de base en dents de scie avec une fréquence $f_{imp}$ = 212,76 Hz, où la durée de l'impulsion de base est $T_{imp}$ = 4,70 ms, le temps $T_{pac\_on}$ est réglé sur 75,20 ms (correspondant au temps de 16 impulsions de base), la pause entre les paquets est réduite à $T_{pac\_off}$ = 21 ms, le temps $T_{tr\_on}$ est réglé sur 384,80 ms (correspondant au temps de 4 paquets) et la pause entre les trains est $T_{tr\_off}$ = 370 ms ;
- programme 6 : impulsion de base en dents de scie avec une fréquence $f_{imp}$ = 231,48 Hz, où la durée de l'impulsion de base est $T_{imp}$ = 4,32 ms, le temps $T_{pac\_on}$ est réglé sur 60,48 ms (correspondant au temps de 14 impulsions de base), la pause entre les paquets est $T_{pac\_off}$ = 16,00 ms, le temps $T_{tr\_on}$ est réglé sur 305,92 ms (correspondant au temps de 4 paquets) et la pause entre les trains est $T_{tr\_off}$ = 350 ms ;
- programme 7 : impulsion de base en dents de scie avec une fréquence $f_{imp}$ = 212,76 Hz, où la durée de l'impulsion de base est $T_{imp}$ = 4,70 ms, le temps $T_{pac\_on}$ est réglé sur 56,40 ms (correspondant au temps de 12 impulsions de base), la pause entre les paquets est $T_{pac\_off}$ = 6,60 ms, le temps $T_{tr\_on}$ est réglé sur 252,00 ms (correspondant au temps de 4 paquets) et la pause entre les trains est $T_{tr\_off}$ = 220 ms ;
- programme 8 : impulsion de base en dents de scie avec une fréquence $f_{imp}$ = 231,48 Hz, où la durée de l'impulsion de base est $T_{imp}$ = 4,32 ms, le temps $T_{pac\_on}$ est réglé sur 43,20 ms (correspondant au temps de 10 impulsions de base), la pause entre les paquets est $T_{pac\_off}$ = 2,44 ms, le temps $T_{tr\_on}$ est réglé sur 183,00 ms (correspondant au temps de 4 paquets) et la pause entre les trains est $T_{tr\_off}$ = 176 ms ; et
- programme 9 : impulsion de base en dents de scie avec une fréquence $f_{imp}$ = 189,75 Hz, où la durée de l'impulsion de base est $T_{imp}$ = 5,27 ms, le temps $T_{pac\_on}$ est réglé sur 26,35 ms (correspondant au temps de 5 impulsions de base), la pause entre les paquets est réglée sur $T_{pac\_off}$ = 16,85 ms, le temps $T_{tr\_on}$ est réglé sur 1728,00 ms (correspondant au temps de 40 paquets) et la pause entre les trains est $T_{tr\_off}$ = 1600 ms.

10. Système de traitement thérapeutique selon la revendication 8 ou la revendication 9, dans lequel lesdites données identifiant un programme de traitement à exécuter comprennent un numéro de programme ou les données de temporisation respectives.

11. Système de traitement thérapeutique selon l'une des revendications précédentes 8 à 10, dans lequel ladite unité de traitement numérique (220a) supplémentaire est configurée pour :

- recevoir des données identifiant une application de traitement ;
- déterminer une séquence d'une pluralité de programmes de traitement en fonction desdites données identifiant une application de traitement ;
- lancer l'exécution d'un premier programme de traitement de ladite séquence de programmes de traitement en envoyant à ladite unité de traitement numérique (220b) des données identifiant ledit premier programme de traitement ;
- attendre (1006, 1008) qu'une durée de programme de traitement donnée soit écoulée ; et
- sélectionner le programme de traitement suivant de ladite séquence de programmes de traitement et lancer l'exécution dudit programme de traitement suivant de ladite séquence de programmes de traitement en envoyant (1002, 1004) à ladite unité de traitement numérique (220b) des données identifiant ledit programme de traitement suivant.

12. Système de traitement thérapeutique selon la revendication 11, dans lequel ladite unité de traitement numérique (220a) supplémentaire est configurée pour recevoir lesdites données identifiant une application de traitement via au moins une parmi : une interface utilisateur (50), et une interface de communication (224a) configurée pour échanger des données avec un système de traitement (10).

13. Système de traitement thérapeutique selon l'une des revendications précédentes 8 à 12, dans lequel ladite unité de traitement numérique (220b) est un FPGA programmable via des données de programme respectives stockées dans une première mémoire non volatile (222b), et/ou ladite unité de traitement numérique (220a) supplémentaire est un microprocesseur programmable via un micrologiciel respectif stocké dans une deuxième mémoire non volatile (222a), et dans lequel ladite unité de traitement numérique (220a) supplémentaire est configurée pour :

   - recevoir de nouvelles données de programme pour ladite unité de traitement numérique (220b) et stocker lesdites nouvelles données de programme dans ladite première mémoire non volatile (222b) ; et/ou
   - recevoir un nouveau micrologiciel pour ladite unité de traitement numérique (220a) supplémentaire et stocker ledit nouveau micrologiciel dans ladite deuxième mémoire non volatile (222b).

14. Système de traitement thérapeutique selon l'une des revendications précédentes, dans lequel ledit circuit de traitement numérique (220b) comprend un générateur d'impulsions (2238) configuré pour générer un signal pulsé ($PWM_A$) configuré pour être appliqué à un transducteur acoustique, lumineux et/ou vibratoire, dans lequel ledit générateur d'impulsions (2238) est configuré pour régler ledit signal pulsé ($PWM_A$) sur haut lorsque ladite deuxième valeur numérique ($S_D$) est supérieure à un seuil, et sur bas lorsque ladite deuxième valeur numérique ($S_D$) est égale ou inférieure audit seuil, ledit signal pulsé ($PWM_A$) ayant une forme d'onde carrée synchronisée avec ledit courant d'alimentation ($i_{out}$) selon ladite fréquence ($f_{imp}$) donnée de ladite impulsion de base (I) périodique, le cycle de commutation ($T_{pac}$) dudit deuxième signal PWM ($PWM_P$) et le cycle de commutation ($T_{tr}$) dudit troisième signal PWM ($PWM_{Tr}$).

15. Système de traitement thérapeutique selon l'une des revendications précédentes, dans lequel ledit appareil (20a) est configuré pour prendre en charge un mode de fonctionnement total et/ou local, dans lequel :

   - dans ledit mode de fonctionnement total, une première antenne (30) est connectée audit appareil (20a), ladite première antenne (30) étant une antenne plane ayant une longueur entre 120 cm et 250 cm, de préférence entre 150 cm et 200 cm, et une largeur entre 40 cm et 120 cm, de préférence entre 60 cm et 90 cm ; et
   - dans ledit mode de fonctionnement local, une deuxième antenne (30) est connectée à l'appareil (20a), ladite deuxième antenne (30) étant une antenne plane ayant une longueur entre 20 cm et 120 cm, de préférence, entre 20 cm et 60 cm, et une largeur entre 20 cm et 60 cm, ou une antenne modélisée anatomiquement.

16. Système de traitement thérapeutique selon la combinaison des revendications 9, 12 et 15, dans lequel ladite séquence d'une pluralité de traitements comprend au moins un parmi :

   - par exemple, pour traiter la sclérose en plaques, une application de traitement total consistant en une séquence des programmes de traitement 1, 3 et 6 et/ou une application de traitement local consistant en une séquence des programmes de traitement 3 et 6 ;
   - par exemple, pour traiter la maladie de Parkinson, une application de traitement total consistant en une séquence des programmes de traitement 3, 6 et 3 et/ou un premier traitement local consistant en une séquence des programmes de traitement 3 et 6 et/ou une deuxième application de traitement local consistant en une séquence des programmes de traitement 8 et 8 ;
   - par exemple, pour traiter la maladie d'Alzheimer ou démence sénile, une application de traitement total consistant en une séquence des programmes de traitement 3, 6 et 9 et/ou une application de traitement local consistant en une séquence des programmes de traitement 3 et 6 ;
   - par exemple, pour traiter les cas d'état post-ictal, une application de traitement total consistant en une séquence des programmes de traitement 3, 3 et 6 et/ou une application de traitement local consistant en une séquence des programmes de traitement 6 et 6 ;
   - par exemple, pour traiter la fibromyalgie ou le syndrome de fatigue chronique, une application de traitement total consistant en une séquence des programmes de traitement 3, 6 et 9 et/ou une application de traitement local consistant en une séquence des programmes de traitement 6 et 8 ; et
- par exemple, pour traiter le système Psycho-Neuro-Endocrine-Immunologie (P.N.E.I.), une application de traitement total consistant en une séquence des programmes de traitement 9, 6 et 9 et/ou une application de traitement local consistant en une séquence des programmes de traitement 3 et 6.

FIG. 1

FIG. 2

# FIG. 3

# FIG. 4

# FIG. 5

# FIG. 6

# FIG. 7

# FIG. 8

# FIG. 9

EP 4 140 035 B1

FIG. 10

## FIG. 11A

## FIG. 11B

FIG. 12A

# FIG. 12B

FIG. 13

FIG. 14

## FIG. 15

# FIG. 16

# FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3160582 A1
- WO 2012172504 A1

- EP 3115999 A1

**Non-patent literature cited in the description**

- PID controller. *Wikipedia page,* 19 April 2020

- Discrete implementation. ADSP-21990: Implementation of PI Controllers". Analog Devices Inc, December 2001